# EUROPEAN PATENT SPECIFICATION

(11) **EP 3 191 517 B1**
(45) Date of publication and mention of the grant of the patent: **04.11.2020**
(21) Application number: 15762562.5
(22) Date of filing: 09.09.2015
(51) Int. Cl.: C07K 16/28, A61P 35/00

(54) **CROSS REACTIVE SIGLEC ANTIBODIES**
KREUZREAKTIVE SIGLEC-ANTIKÖRPER
ANTICORPS ANTI-SIGLEC À RÉACTION CROISÉE

(30) Priority: 10.09.2014 US 201462048292 P
(43) Date of publication of application: 19.07.2017
(73) Proprietor: Innate Pharma, 13009 Marseille (FR)
(72) Inventor: CORNEN, Stéphanie, F-13009 Marseille (FR); ROSSI, Benjamin, F-13008 Marseille (FR); WAGTMANN, Nicolaï, F-13260 Cassis (FR)
(74) Representative: Gallois, Valérie
(86) International application number: PCT/EP2015/070550
(87) International publication number: WO 2016/038064

(56) References cited:
- R&d Systems: "Human Siglec-7/CD328 Antibody", , 7 August 2014 (2014-08-07), pages 1-2, XP055227710, Retrieved from the Internet: URL:https://resources.rndsystems.com/pdfs/ datasheets/af1138.pdf [retrieved on 2015-11-11]
- Camilla Jandus ET AL: "Interactions between Siglec-7/9 receptors and ligands influence NK cell-dependent tumor immunosurveillance", JOURNAL OF CLINICAL INVESTIGATION, vol. 124, no. 4, 24 February 2014 (2014-02-24), pages 1810-1820, XP055342636, GB ISSN: 0021-9738, DOI: 10.1172/JCI65899
- Jason E Hudak ET AL: "Glycocalyx engineering reveals a Siglec-based mechanism for NK cell immunoevasion-Supplementary information", NATURE CHEMICAL BIOLOGY, vol. 10, no. 1, 24 November 2013 (2013-11-24), pages S1-S21, XP055372305, Basingstoke ISSN: 1552-4450, DOI: 10.1038/nchembio.1388

## Description

### FIELD OF THE INVENTION

This invention relates to agents that bind multiple Siglecs, including antibodies that neutralize the inhibitory activity of multiple Siglecs in lymphocytes. Such agents can be used for the treatment of cancers or infectious disease.

### BACKGROUND OF THE INVENTION

NK cells are mononuclear cell that develop in the bone marrow from lymphoid progenitors, and morphological features and biological properties typically include the expression of the cluster determinants (CDs) CD16, CD56, and/or CD57; the absence of the alpha/beta or gamma/delta TCR complex on the cell surface; the ability to bind to and kill target cells that fail to express "self" major histocompatibility complex (MHC)/human leukocyte antigen (HLA) proteins; and the ability to kill tumor cells or other diseased cells that express ligands for activating NK receptors. NK cells are characterized by their ability to bind and kill several types of tumor cell lines without the need for prior immunization or activation. NK cells can also release soluble proteins and cytokines that exert a regulatory effect on the immune system; and can undergo multiple rounds of cell division and produce daughter cells with similar biologic properties as the parent cell. Normal, healthy cells are protected from lysis by NK cells.

Based on their biological properties, various therapeutic and vaccine strategies have been proposed in the art that rely on a modulation of NK cells. However, NK cell activity is regulated by a complex mechanism that involves both stimulating and inhibitory signals. Briefly, the lytic activity of NK cells is regulated by various cell surface receptors that transduce either positive or negative intracellular signals upon interaction with ligands on the target cell. The balance between positive and negative signals transmitted via these receptors determines whether or not a target cell is lysed (killed) by a NK cell. NK cell stimulatory signals can be mediated by Natural Cytotoxicity Receptors (NCR) such as NKp30, NKp44, and NKp46; as well as NKG2C receptors, NKG2D receptors, certain activating Killer Ig-like Receptors (KIRs), and other activating NK receptors (Lanier, Annual Review of Immunology 2005;23:225-74). NK cell inhibitory signals can be mediated by receptors like Ly49, CD94/NKG2-A, as well as certain inhibitory KIRs, which recognize major histocompatibility complex (MHC) class I-molecules (Kärre et al., Nature 1986;319:675-8; Öhlén et al, Science 1989;246:666-8). These inhibitory receptors bind to polymorphic determinants of MHC class I molecules (including HLA class I) present on other cells and inhibit NK cell-mediated lysis.

The lytic activity of NK cells can also be regulated by siglec polypeptides. Siglecs (sialic-acid-binding immunoglobulin-like lectins) are a subset of I-type lectins that bind to si-aloglycans and are predominantly expressed on cells of the hematopoietic system in a manner dependent on cell type and differentiation. Whereas sialic acid is ubiquitously expressed, typically at the terminal position of glycoproteins and lipids, only very specific, distinct sialoglycan structures are recognized by individual Siglec receptors, depending on identity and linkage to subterminal carbohydrate moieties. Siglecs have only low general affinity to the common mammalian sialoside structures containing the N-acetylneuraminic acid (Neu5Ac) α2-6 and α2-3 linkages.

Siglecs are generally divided into two groups, a first subset made up of Siglec-1, -2, -4 and -15, and the CD33-related group of Siglecs which includes Siglec-3, -5, -6, -7, -8, -9, - 10, -11, -12, -14 and -16. The CD33-related Siglecs are characterized, inter alia, by low evolutionary conservation and rapidly evolving sequence by multiple mechanisms.

Siglec-7 (CD328), a type 1 trans-membrane protein first cloned and characterized in 1999 by the Moretta group in Genoa and belonging to the human CD33-related Siglec receptors, is characterized by a sialic acid binding N-terminal V-set Ig domain, two C2-set Ig domains and an intracytoplasmic region containing one immune-receptor tyrosine based inhibitory motif (ITIM) and one ITIM-like motif. Siglec-7 is constitutively expressed on NK cells, dendritic cells, monocytes, neutrophils and CD8+ T cells. The extracellular domain of this receptor preferentially binds a (2,8)-linked disialic acids and branched α 2,6-sialyl residues, such as those displayed by ganglioside GD3.

Siglec-9 (CD329) was characterized in 2000 by the Varki group (see, e.g., Angata et al. J Biol Chem 2000; 275:22127-22135) and is expressed on monocytes, neutrophils, dendritic cells, CD34+ cells, CD8+ T cells and NK cells. Siglec-9 (as well as Siglec-8) have been found to have differential specificity for sialoside ligands that contain both sialic acid and sulfate, with the position of the sulfate being an important determinant of specificity. Siglec-9 has been found to bind MUC16 that is overexpressed on cancer cells. Like Siglec-7, Siglec 9 also contains a sialic acid binding N-terminal V-set Ig domain, two C2-set Ig domains and an intracytoplasmic region containing one immune-receptor tyrosine based inhibitory motif (ITIM) and one ITIM-like motif. N-terminal V-set Ig domain of human Siglec-9 shares an overall amino acid sequence identity of about 77% with N-terminal V-set Ig domain of human Siglec-7, and these two siglecs display different sialic acids binding specificities.

Various antibodies against each of Siglec-7 and Siglec-9 have been described. For example, European Patent 1238282B1 (Moretta et al) and Vitale et al. ((1999) Proc. Nat. Acad. Sci. 96(26):15091-96) refers to the murine anti-siglec-7 antibody QA79. Falco et al. (1999) J. Exp. Med. 190:793-801 report antibody Z176. An anti-siglec-9 antibody has also been reported; clone E10-286 available from BD Biosciences. Jandus et al. 2014 J. Clin. Invest. 124(4): 1810-1820 makes use of anti-Siglec-7 antibody Z176 and anti-Siglec-9 antibody E10-286, while Hudak et al. (2013) Nature 10(1): S1-S21 makes use of anti-Siglec-7 antibody 6-434 (BioLegend), anti-Siglec-7 antibody 7.7 (BioLegend), anti-Siglec-9 antibody 191240 (BioLegend) and anti-Siglec-9 antibody K8 (BioLegend). However these antibodies do not bind both Siglec-7 and Siglec-9. Finally, R&D Systems "Human Siglec-7/CD328 Antibody, AF1138", 2014-08-07, pages 1-2, XP055227710 refers to a polyclonal antibody composition, and without a teaching or suggestion that such antibody can neutralize human Siglec-7.

Despite the interest in Siglec-7 and -9, no therapeutic agents targeting these receptors have been developed. There is therefore a need for agents that target these receptors for use in treating diseases such as cancer.

### SUMMARY OF THE INVENTION

The scope of the present invention is defined by the claims and any information that does not fall within the claims is provided for information only.

As shown herein, NK cells can express both the inhibitory Siglec-7 and the inhibitory Siglec-9 protein. At the same time, it has been shown that tumor cells can express the natural ligands (glycans) for Siglec-7 and for Siglec-9. Consequently, a therapeutic agent that inhibits of one Siglec but not the other may not be maximally efficient in neutralizing Siglec-mediated restriction of the activity of NK and/or other immune cells. Inhibition of both Siglec 7 and 9 can therefore be advantageous. However, Siglec-9 shares an overall amino acid sequence identity of only about 77% with N-terminal V-set Ig domain of human Siglec-7. Moreover, these two siglecs display different sialic acids binding specificities.

In one aspect, the present disclosure provides antibodies that specifically bind to two or more inhibitory human Siglec polypeptides (e.g. Siglec polypeptides as expressed at the surface of a cell). In one aspect, the antibodies furthermore are capable of inhibiting (e.g., neutralizing antibodies) the inhibitory activity of at least two such Siglecs, notably by blocking the sialic acid ligand-induced inhibitory activity of such Siglec polypeptides in immune cells. The antibodies can bind to a common determinant present on the two or more Siglecs. Optionally, the two or more Siglec polypeptides are CD33-related Siglec polypeptides. In one aspect, the two or more Siglec polypeptides are Siglec-7 and Siglec-9. In one aspect, the antibodies furthermore do not substantially bind any of Siglecs-3, -5, -6, -8, -10, -11 and-12. In one aspect, the antibodies furthermore do not substantially bind any of Siglecs-14 and -16.

In any aspect herein, the antibodies can, for example, block the interactions between each of the Siglecs and a sialoside-containing ligand(s) thereof (e.g., a natural ligand) and/or block the Siglec activity (inhibitory signal) induced by a sialoside-containing ligand thereof. In any aspect herein, the antibodies can, for example, block the interactions between each of the Siglecs and a sialoside-free ligand(s) thereof (e.g., a natural protein ligand that lacks sialosides) and/or block the Siglec activity (inhibitory signal) induced by a sialoside-containing ligand thereof.

In one aspect, the present disclosure provides an antibody that specifically bind to a human Siglec-7 and/or -9 polypeptide and is capable of inihibiting (e.g., a neutralizing antibody) the inhibitory activity of such Siglec(s) in immune cells by blocking the inhibitory activity of such Siglec polypeptide(s) induced by a sialoside-free protein ligand. In one aspect, the disclosure provides an antibody that specifically bind to a human Siglec-7 polypeptide (with or without the ability to bind to any other Siglec polypeptides), and which is capable of blocking the interactions between Siglec-7 and a sialoside-free ligand(s) of Siglec-7. In one aspect, the disclosure provides an antibody that specifically bind to a human Siglec-9 polypeptide (with or without the ability to bind to any other Siglec polypeptides), and which is capable of blocking the interactions between Siglec-9 and a sialoside-free ligand(s) of Siglec-9.

Fragments and derivatives of such antibodies are also provided. In one aspect, the antibody comprises an antigen-binding domain (e.g., a single antigen binding domain, a domain made up of a heavy and a light chain variable domain, etc.) capable of binding to said two or more human Siglecs (i.e. cross-reactive with the two or more Siglecs).

In one aspect, the disclosure is related to an antigen binding domain (isolated or comprised in an isolated polypeptide, for example and antibody) that is capable of specifically binding to a human Siglec-7 polypeptide and to a human Siglec-9 polypeptide. In one aspect, the antigen binding domain does not substantially bind any of Siglecs-3, -5, -6, -8, -10, - 11 and -12.

The disclosure is also related, inter alia, to an antigen binding domain or antibody that specifically binds to and neutralizes the inhibitory activity of both of the human inhibitory receptors Siglec-7 and Siglec-9 (a neutralizing anti-Siglec-7/9 antibody). In one aspect, the antigen binding domain or antibody does not substantially bind any of Siglecs-3, -5, -6, -8, - 10, -11 and -12. The neutralizing anti-Siglec-7/9 antibody relieves the inhibitory activity exerted by Siglec-7 and -9 in immune cells, enhancing the ability of lymphocytes to effectively recognize and/or eliminate cancer cells that express sialic acid ligands of Siglec-7 and/or sialic acid ligands of Siglec-9. The cross-reactive and neutralizing antibody reduces the ability of cancer cells to escape lysis due to expression of one or the other types of ligand, and they therefore enhance tumor surveillance by the immune system. Siglec-9 is expressed selectively on CD56^{dim} NK cells, while siglec-7 is expressed on CD56^{dim} and CD56^{bright} NK cells. CD56^{dim} NK cells (CD56^{dim}CD16⁺KIR⁺) represent about 90% of peripheral blood and spleen NK cells, express perforin and granzymes, and are the major cytotoxic subset, whereas CD56^{bright} NK cells (CD56^{bright}CD16^{dim/-}KIR⁻) constitute the majority of NK cells in lymph nodes and tonsils and, upon activation, primarily respond with cytokine production.

In one aspect, provided is an antibody that can bind both Siglec-7 and Siglec-9 and which can neutralize both Siglec-7 and Siglec-9-mediated inhibition of lymphocytes (e.g. NK cell, CD8+ T cell) cytotoxicity. In one aspect, the antibody increases lymphocyte activation in the presence of a target cell (e.g. a cell that expresses a ligand of Siglec-7 and a ligand of Siglec-9, a tumor cell). In one aspect, the antibody increases NK cell and/or CD8+ T cell activation. In one aspect, increased activation or neutralization of inhibition of cytotoxicity is assessed by increase in a marker of cytotoxicity/cytotoxic potential, e.g. CD107 and/or CD137 expression (mobilization). The Siglec-7 may comprise an amino acid sequence of SEQ ID NOS: 1 or 52. The Siglec-9 may comprise an amino acid sequence of SEQ ID NOS: 2 or 53.

In one aspect, the antibody is a tetrameric (e.g., full length, F(ab)'2 fragment) antibody that is cross-reactive with two or more Siglecs, e.g., Siglec-7 and Siglec-9, and can bind a common epitope present on the extracellular domain of the two or more Siglecs, i.e. in bivalent fashion. In one aspect, the antibody can bind each of the two or more Siglecs (e.g. Siglec-7 and -9) bivalently. In another aspect, the antibody binds to a Siglec in monovalent manner and lacks agonist activity at each Siglec, e.g., Siglec-7 and Siglec-9. In one aspect, the antibody that binds each Siglec in monovalent manner is a Fab fragment. In any of the aspects herein, the antibody binds to each Siglec in monovalent or bivalent manner is free of agonist activity at each Siglec, e.g., Siglec-7 and Siglec-9. For therapeutic use, an antibody is preferably a non-depleting antibody. Optionally the antibody comprises and Fc domain capable of be bound by the human neonatal Fc receptor (FcRn) but which substantially lacks binding, via its Fc domain, to a human FcyR (e.g. CD16).

In one aspect, the antibody comprises one or more (e.g. two) antigen binding domain that binds to both Siglec-7 and Siglec-9. In one specific aspect, the antibody is a tetrameric, optionally full-length, antibody that comprises a two identical antigen binding domains (optionally, two heavy and light chain variable region pairs), and that binds and neutralizes the inhibitory activity of Siglec-7 and Siglec-9, comprises an Fc domain capable of be bound by the human neonatal Fc receptor (FcRn) and that substantially lacks binding to a human FcyR (e.g. CD16).

In any of the aspects herein, upon binding to a Siglec on a human lymphocyte, the monoclonal antibody has the ability to enhance or reconstitute lysis of a target human cell bearing a sialic acid ligand of the Siglec on the target cell surface, and/or has the ability to increase lymphocyte activation (e.g., as determined by an increase in CD107 and/or CD137 expression on a lymphocyte), when said target cell comes into contact with said lymphocyte (e.g. an effector lymphocyte, an NK or a CD8+ T cell). In one aspect, provided is an antibody that neutralizes two or more Siglecs expressed by lymphocytes (e.g., two Siglecs expressed by the same subset of lymphocytes or expressed by different subsets of lymphocytes). In one aspect, provided is an antibody neutralizes a first Siglec expressed by a first subsets of lymphocytes, and that neutralizes a second Siglec expressed by a second subset of lymphocytes. The first and second subset of lymphocytes (e.g. NK cells, CD8+ T cells) can for example be characterized by different cell surface markers or different functional properties, or the ability to lyse or recognize (e.g., be activated by) different target cells. In one aspect, the antibody reduces (blocks) binding of a Siglec to a sialoside ligand thereof (e.g. a ligand present on tumor cells).

In one aspect, the present disclosure provides an antibody that is a monoclonal antibody or a fragment thereof characterized by:
a) specifically binding to a first CD33-related Siglec, and when bound to the first CD33-related Siglec on a human lymphocyte, neutralizing said Siglec-mediated inhibition of NK or T cell cytotoxicity when the human immune cell comes into contact with a target cell bearing a ligand of the first CD33-related Siglec on the target cell surface;
b) specifically binding to a second CD33-related Siglec, and when bound to the second CD33-related Siglec on a human lymphocyte, neutralizing said Siglec-mediated inhibition of NK or T cell cytotoxicity when the human immune cell comes into contact with a target cell bearing a ligand of the second CD33-related Siglec on the target cell surface; and
c) not substantially binding (e.g. via an Fc domain of the antibody) to a human Fcγ receptor (e.g. CD16). In one aspect, the antibody is a full length antibody comprising a human Fc domain. In one aspect, the lymphocyte is an NK cell. In one aspect, the lymphocyte is a cytotoxic CD8+ T cell. In one aspect, the ligand of the first and/or second CD33-related Siglec is a sialic acid or molecule comprising a sialic acid. In one aspect, the first CD33-related Siglec is Siglec-7. In one aspect, the second CD33-related Siglec is Siglec-9.

In one aspect, the present disclosure provides an antibody that is a monoclonal antibody or a fragment thereof characterized by:
a) specifically binding to Siglec-7, and when bound to Siglec-7 on a human lymphocyte, causing (e.g. increasing the ability of) said lymphocyte to lyse a target human cell bearing a ligand of Siglec-7 on the target cell surface, when said target cell comes into contact with said lymphocyte;
b) specifically binding to Siglec-9, and when bound to Siglec-9 on a human lymphocyte, causing (e.g. increasing the ability of) said lymphocyte to lyse a target human cell bearing a ligand of Siglec-9 on the target cell surface, when said target cell comes into contact with said lymphocyte; and
c) not substantially binding (e.g. via an Fc domain of the antibody) to a human Fcy receptor (e.g. CD16). In one aspect, the antibody is a full length antibody comprising a human Fc domain. In one aspect, the lymphocyte is an NK cell. In one aspect, the lymphocyte is a CD8+ T cell. In one aspect, the ligand of the first and/or second CD33-related Siglec is a sialic acid or molecule comprising a sialic acid.

In one aspect, the present disclosure provides an antibody that is a monoclonal antibody or a fragment thereof characterized by:
a) specifically binding to Siglec-7, and when bound to Siglec-7 on a human lymphocyte, causing an increase in lymphocyte activation or cytotoxicity in the presence of a target cell bearing a ligand of Siglec-7 on the target cell surface, when said target cell comes into contact with said lymphocyte;
b) specifically binding to Siglec-9, and when bound to Siglec-9 on a human lymphocyte, causing an increase in lymphocyte activation or cytotoxicity in the presence of a target cell bearing a ligand of Siglec-9 on the target cell surface, when said target cell comes into contact with said lymphocyte; and
c) not binding (e.g. via an Fc domain of the antibody) to a human Fcγ receptor (e.g. CD16). In one aspect, the antibody is a full length antibody comprising a human Fc domain. In one aspect, the lymphocyte is an NK cell. In one aspect, the lymphocyte is a CD8+ T cell. In one aspect, the ligand of the first and/or second CD33-related Siglec is a sialic acid or molecule comprising a sialic acid. In one aspect, increased activation or neutralization of inhibition of cytotoxicity is assessed by increase in a marker of cytotoxicity/cytotoxic potential, e.g. CD107 and/or CD137 expression (mobilization).

In any of the aspects herein, the antibody blocks the inhibitory signalling by a Siglec triggered by a sialoside ligand of the Siglec. In any of the aspects herein, the antibody blocks binding of a Siglec to a sialoside ligand of the Siglec.

In any of the aspects herein, the ligand of a Siglec is a natural ligand, e.g. a sialic acid ligand (a ligand comprising a sialic acid). Sialic acids, a family of nine-carbon acidic monosaccharides, are typically found to be terminating branches of N-glycans, O-glycans, and glycolipids. Siglecs are belived to recognize many aspects of the sialic acid molecule, like the acid sialic linkage from the 2-position, the arrangements of sialic acids and their way of presentation. In any of the aspects herein, the ligand of a Siglec comprises mainly a 5-N-acetylneuraminic acid (Neu5Ac) derivative, and can comprises other sialic acid derivatives, like 5-N-glycolylneuraminic acid (Neu5Gc) derivatives.. In one aspect, the ligand of Siglec- 9 and/or Siglec-7 is a sialic acid present on a glycoprotein (e.g. a mucin) or a glycolipid. In one aspect, the ligand of Siglec-7 comprises a α2,8-linked disialic acid presented on b-series gangliosides, e.g., GD2, GD3 and GT1b. In one aspect, the ligand of Siglec-7 comprises an internally branched alpha2,6-linked disialic gangliosides, e.g., DSGb5. In one aspect, the ligand of Siglec-9 is a ligand present on, or comprises, a mucin, e.g. MUC1. In one aspect, the ligand of Siglec-9 is a sialoglycan ligand that contains both sialic acid and sulfate.

In one aspect, provided is an antibody that binds to a common determinant present on an extracellular domain of as first and a second human CD33-related Siglec. Optionally the antibody blocks the inhibitory activity of each of said first and second Siglecs, e.g. when a cell (for example an NK cell) expressing the Siglec comes into contact with a target human cell bearing a sialic acid ligand of the Siglec. In one aspect, the first CD33-related Siglec is Siglec-7. In one aspect, the second CD33-related Siglec is Siglec-9. The antibody can thereby increase NK and/or T cell activation and/or cytotoxicity.

In one aspect, provided is an antibody binds to a common determinant present on Siglec-7 and on Siglec-9. Optionally, the common determinant is not present on one or more other Siglecs, e.g. one or more of (or all of) Siglecs-3, -5, -6, -8, -10, -11 and-12.

In any of the aspects herein, the antibody binds to an extracellular domain of the Siglec. In any of the aspects herein, the antibody binds at least partially within or near the sialic acid binding domain of the Siglec.

In any of the aspects herein, upon binding to a Siglec on a human lymphocyte (e.g. NK cell), the monoclonal antibody has the ability to reconstitute lysis of a target human cell bearing a sialic acid ligand of the Siglec on the target cell surface, when said target cell comes into contact with said lymphocyte.

In any of the aspects herein, the antibody has a KD of less than 10⁻⁸ M, preferably less than 10⁻⁹ M for binding to each of the first and second Siglec polypeptide (e.g. human Siglec-7 and human Siglec-9). Insofar as the Siglec-7 and -9 binding sites are believed to generally masked at the cellular surface due to cis interactions with abundantly expressed low affinity sialic acids, *trans* interactions can occur with antibodies expressing higher affinity than the ligands that compete with *cis.* In one aspect, the neutralizing anti-Siglec antibody is capable of displacing the binding of a sialoside ligand to a Siglec (e.g. Siglec-7 and/or Siglec-9).

The disclosure also provides a human or humanized antibody or antibody fragment, or a derivative thereof, which has any of the foregoing properties, alone or in any suitable combination.

In one aspect, the antibody is a monoclonal antibody. In one aspect, the antibody is an IgG1, IgG2, IgG3, or IgG4 antibody. For example, the antibody may be an antibody comprising an Fc domain of human IgG4 isotype or an antibody comprising an Fc domain of any human IgG isotype (e.g. IgG1, IgG2, IgG3, or IgG4) modified to reduce binding between the Fc domain and an Fcγ receptor (e.g. CD16). In one aspect, an antibody that binds to two or more Siglecs (e.g.Siglec-7 and Siglec-9) expressed by an NK or T cell does not lead, directly or indirectly, to the depletion of NK cells expressing such a Siglec polypeptide (e.g. do not lead to a 10%, 20%, 50%, 60% or greater elimination or decrease in number of Siglec+ NK or T cells). Preferably, the antigen-binding compound does not comprise an Fc domain capable of inducing antibody mediated cellular cytoxicity (ADCC) and/or CDC; optionally the antigen-binding compound does not comprise an Fc domain capable of substantially binding to a FcγRIIIA (CD16) polypeptide, or comprises an Fc domain not capable of substantially binding to a FcγRIIIA (CD16) polypeptide; preferably the antigen-binding compound lacks an Fc domain (e.g. lacks a CH2 and/or CH3 domain) or comprises an Fc domain of IgG2 or IgG4 isotype, optionally an Fc domain of IgG4 isotype comprising a stabilizing mutation to decrease formation of half-antibodies such as a S241P mutation; optionally the antigen-binding compound consists of or comprises a Fab, Fab', Fab'-SH, F (ab') 2, Fv, a diabody, single-chain antibody fragment, or a multispecific antibody comprising multiple different antibody fragments. Preferably the antigen-binding compound is not linked to a toxic moiety. Preferably the antibody does not act as an agonist of the Siglec polypeptides, e.g. the antibody is optionally not capable of causing sufficient cross-linking, in vivo, of Siglec polypeptide on an NK cell so as to cause signalling by the Siglec.

Provided in one aspect are monoclonal antibodies that compete for binding to an epitope on Siglec-7 and/or Siglec-9 bound by 3A11, 1H9 and/or 2B4, (e.g., that competes for binding to an epitope on a Siglec-7 and/or Siglec-9 polypeptide with an antibody having the heavy and light chain CDRs or variable regions of any of 3A11, 1H9 or 2B4).

In one aspect, provided is an antigen-binding compound that binds the same epitope and/or competes for binding to a Siglec-7 and/or Siglec-9 polypeptide with monoclonal antibodies 3A11, 1H9 or 2B4 (e.g., that competes for binding to a Siglec-7 and/or Siglec-9 polypeptide expressed by a cell with an antibody having the heavy and light chain CDRs or variable regions of any of 3A11, 1H9 or 2B4). In one aspect, provided is antigen-binding compound binds the same epitope and/or competes for binding to a Siglec-7 and/or Siglec-9 polypeptide with an antibody selected from the group consisting of:
(a) an antibody having respectively a VH and VL region of SEQ ID NOS: 3 and 4 (3A11);
(b) an antibody having respectively a VH and VL region of SEQ ID NOS: 21 and 22 (1H9); and
(c) an antibody having respectively a VH and VL region of SEQ ID NOS: 39 and 40 (2B4).

In one aspect, the antibody may have a heavy and/or light chain having one, two or three CDRs of the respective heavy and/or light chain of an antibody selected from the group consisting of antibody 3A11, 1H9 and 2B4.

In one aspect, binding to a Siglec can be specified as being cellular Siglec, where the Siglec is expressed at the surface of a cell, for example a native or modified cellular Siglec, a Siglec expressed by a recombinant host cell, a Siglec expressed by an NK cell, a CD8 T cell, etc.

The disclosure also provides a nucleic acid encoding the human or humanized antibody or antibody fragment having any of the foregoing properties, a vector comprising such a nucleic acid, a cell comprising such a vector, and a method of producing a human anti- Siglec antibody, comprising culturing such a cell under conditions suitable for expression of the anti-Siglec antibody. The disclosure also relates to compositions, such as pharmaceutically acceptable compositions and kits, comprising such proteins, nucleic acids, vectors, and/or cells and typically one or more additional ingredients that can be active ingredients or inactive ingredients that promote formulation, delivery, stability, or other characteristics of the composition (*e.g.,* various carriers). The disclosure further relates various new and useful methods making and using such antibodies, nucleic acids, vectors, cells, organisms, and/or compositions, such as in the modulation of Siglec-mediated biological activities, for example in the treatment of diseases related thereto, notably cancers and infectious disease.

The disclosure also provides methods of producing an antibody which cross-reacts with two or more Siglec gene products, and which optionally further neutralizes the inhibitory activity two or more Siglecs, said method comprising the steps of:
(a) providing a plurality of antibodies that bind a CD33-related Siglec polypeptide,
(b) selecting antibodies (e.g. those of step of (a)) that cross-react with at least two different CD33-related Siglec gene products, and
(c) optionally, selecting antibodies (e.g. those of step (b)) that neutralizes the inhibitory activity of at least two different CD33-related Siglec gene products.

In another aspect, the methods of producing an antibody which cross-reacts with two or more Siglec gene products and further neutralizes the inhibitory activity two or more Siglecs, comprises:
(a) providing a plurality of antibodies that bind a CD33-related Siglec polypeptide,
(b) selecting antibodies (e.g. those of step of (a)) that cross-react with at least two different CD33-related Siglec gene products, and
(c) optionally, selecting antibodies (e.g. those of step (b)) that block the interaction between each of two different CD33-related Siglec gene products and a respective sialic acid ligand thereof.

It will be appreciated that steps (b) and (c) in any of the above methods can be carried out in any desired order.

In one aspect, selecting antibodies (e.g. of step (c)) that neutralizes the inhibitory activity of two different Siglec gene products in any of the above methods can comprise selecting antibodies that potentiate NK cells.

In one aspect, selecting antibodies (e.g. of step (c)) that neutralizes the inhibitory activity of two different Siglec gene products comprises determining whether antibodies neutralize the inhibitory activity of at least one of the two different Siglec gene products, wherein a determination that an antibody neutralizes the inhibitory activity of said at least one Siglec indicates that the antibody is capable of neutralizing the inhibitory activity of two different Siglec gene products. In one aspect, determining whether antibodies neutralize the inhibitory activity of at least one of the two different Siglec gene products comprises assessing whether the antibody causes an increase in a marker of cytotoxicity (e.g. an increase in expression of CD107 and/or CD137) when lymphocytes expressing the Siglec(s) are brought into contact with target cells (e.g. that express ligands of the Siglec(s)). An increase in a marker of cytotoxicity (e.g. an increase in expression of CD107 and/or CD137) indicates that the antibody is capable of neutralizing the inhibitory activity of two different Siglec gene products.

The selection of an antibody that cross-reacts with at least two different Siglec gene products may be achieved by screening the antibody for binding to the two or more different inhibitory Siglec polypeptides (e.g. Siglec-7 and -9). In one aspect, the method further comprises by screening the antibody for binding to one or more additional Siglec polypeptides for which binding is not desired, and selection antibodies that do not bind such additional Siglec polypeptide(s), optionally additional Siglec polypeptide is one or more of (or all of) Siglecs-3, -5, -6, -8, -10, -11 and-12.

In one aspect, the antibodies prepared in step (b) are monoclonal antibodies.

In one aspect, providing a plurality of antibodies in step (a) comprises immunizing a non-human mammal with an immunogen comprising a CD33-related Siglec polypeptide (e.g., Siglec-7 and/or -9), and preparing antibodies from said immunized mammal, wherein said antibodies bind said Siglec polypeptides. The term "preparing antibodies from said immunized animal," as used herein, includes obtaining B-cells from an immunized animal and using those B cells to produce a hybridoma that expresses antibodies, as well as obtaining antibodies directly from the serum of an immunized animal. In one aspect, providing a plurality of antibodies in step (a) comprises producing a library of antibodies, e.g. by phage display.

The CD33-related polypeptide can, in one aspect, be Siglec-7 and/or Siglec-9. The antibodies selected cross-react with at least Siglec-7 and Siglec-9. The antibodies selected cross-react with one or more of (or all of) Siglecs-3, -5, -6, -8, -10, -11 and-12. In one aspect the antibody recognizes a common determinant present on at least two different CD33-related receptor gene products; most preferably the Siglecs are Siglec-7 and Siglec-9. In one aspect, both Siglec-7 and Siglec-9 polypeptides are used for immunization and/or are assessed of binding in the selection of antibodies, either simultaneously or sequentially.

The disclosure also provides an in vitro method for modulating the activity of Siglec-7 and/or Siglec-9-expressing lymphocytes, optionally NK cells and/or CD8+ T cells, the method comprising bringing lymphocytes expressing at their surface Siglec-7 and/or Siglec-9 into contact with an antibody that neutralizes the inhibitory activity of Siglec-7 and Siglec-9.

The disclosure also provides a method of potentiating and/or modulating the activity of lymphocytes (e.g., NK cells, CD8+ T cells) activity in a subject in need thereof, for example a method of potentiating NK cell activity by modulating both CD56^{dim} NK cells (the major cytotoxic subset) and CD56^{bright} NK cells (the majority of NK cells in lymph nodes and tonsils and, upon activation, primarily respond with cytokine production), which method comprises administering to the subject an effective amount of any of the foregoing compositions. In one aspect, the subject is a patient suffering from cancer. For example, the patient may be suffering from a hematopietic cancer, e.g., acute myeloid leukaemia, chronic myeloid leukaemia, multiple myeloma, or non-Hodgkin's lymphoma. Alternatively, the patient may be suffering from a solid tumor, e.g. colorectal cancer, renal cancer, ovarian cancer, lung cancer, breast cancer or malignant melanoma. In another aspect, the subject is a patient suffering from an infectious disease.

### BRIEF DESCRIPTION OF THE DRAWINGS

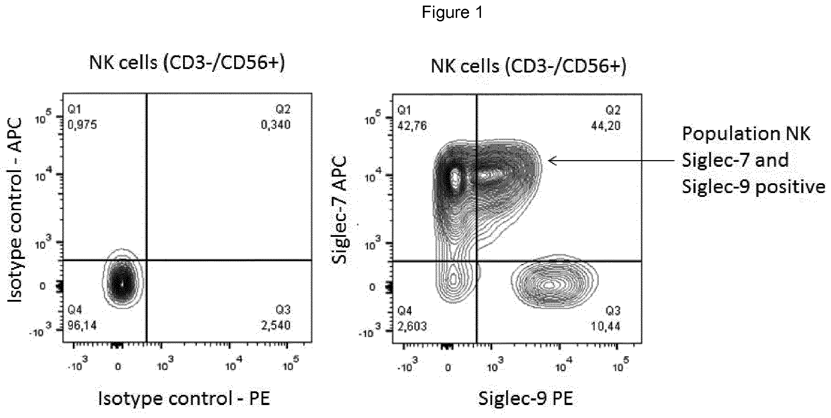

Figure 1 shows binding of anti-Siglec antibodies to NK cells. Siglec MFI:Mean of fluorescence intensity. A significant fraction (about 44%) of NK cells expressed both Siglec-7 and Siglec-9, suggesting that a large proportion of NK cells can be inhibited by each of (or both of) these receptors, as a function of the glycan ligands present, for example on tumor cells.

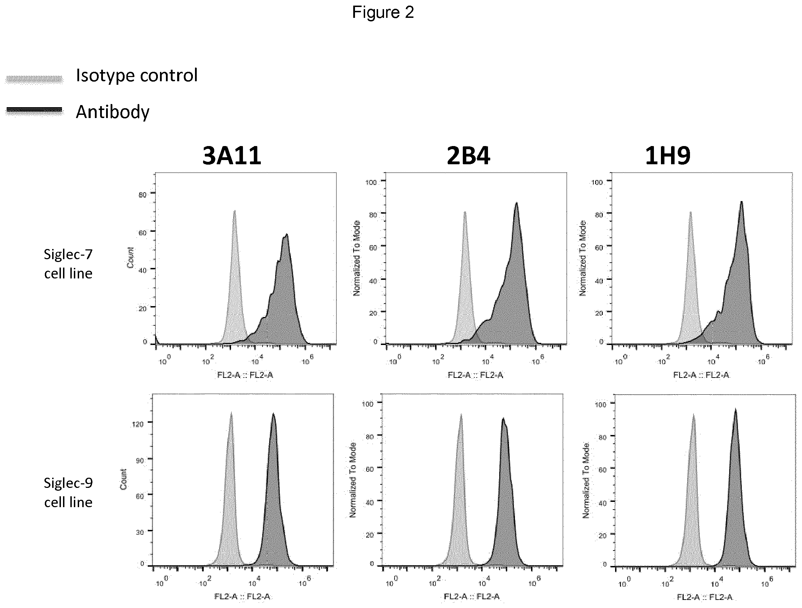

Figure 2 shows results of binding to Siglec-7 and Siglec-9 for mAbs 3A11, 1H9 and 2B4, each of which bound to both human Siglec-7 and Siglec-9-expression cells lines.

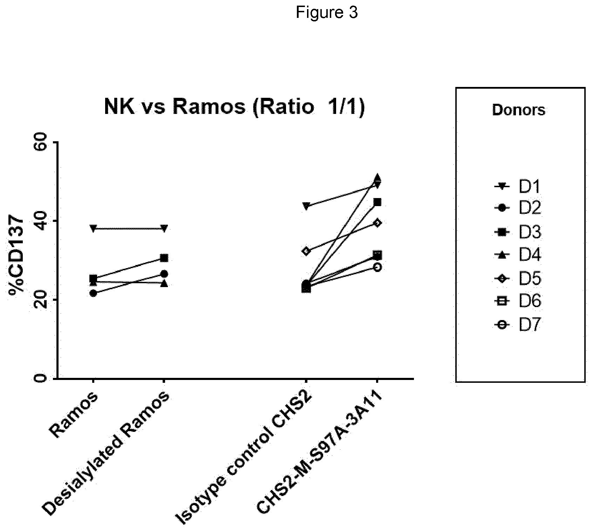

Figures **3** shows CD137 FACS read-outs on NK cells in presence of target cells or desialylated target cells and in presence of 3A11 anti-Siglec-7/9 antibody or isotype control CHS2 antibody at a saturating dose of 10 µg/mL. Each dot represents NK from a healthy volunteer. 3A11 antibody induced an increase of CD137 expression on NK cells in presence of the target cell line Ramos. 3A11 antibody had no effect on CD137 expression on NK cells in absence of target cell line.

### DETAILED DESCRIPTION

### Definitions

As used in the specification, "a" or "an" may mean one or more. As used in the claim(s), when used in conjunction with the word "comprising", the words "a" or "an" may mean one or more than one. As used herein "another" may mean at least a second or more.

Where "comprising" is used, this can optionally be replaced by "consisting essentially of" or by "consisting of".

Human Siglec-7 (shown in Genbank accession number NP_055200.1) is a member of the CD33-related Siglec family (Angata and Varki, Glycobiology 10 (4), 431-438 (2000)). Human Siglec-7 comprises 467 amino acids, having the following amino acid sequence:

Human Siglec-9 (shows in Genbank accession number AAF71455) is a member of the CD33-related Siglec family (Angata and Varki, J. Biol. Chem. 275 (29), 22127-22135 (2000)). Human Siglec-9 comprises 463 amino acids, having the following amino acid sequence:

In the context of the present disclosure, "neutralize Siglec-mediated inhibition of NK cell cytotoxicity", ""neutralize Siglec-mediated inhibition of T cell cytotoxicity" or "neutralize the inhibitory activity of a Siglec,"" refers to a process in which a Siglec (e.g. Siglec-7, Siglec-9) is inhibited in its capacity to negatively affect intracellular processes leading to lymphocyte responses such as cytokine release and cytotoxic responses. This can be measured for example in a standard NK- or T-cell based cytotoxicity assay, in which the capacity of a therapeutic compound to stimulate killing of sialic-acid ligand positive cells by Siglec positive lymphocytes is measured. In one aspect, an antibody preparation causes at least a 10% augmentation in the cytotoxicity of a Siglec-restricted lymphocyte, optionally at least a 40% or 50% augmentation in lymphocyte cytotoxicity, or optionally at least a 70% augmentation in NK cytotoxicity, and referring to the cytotoxicity assays described. In one aspect, an antibody preparation causes at least a 10% augmentation in cytokine release by a Siglec-restricted lymphocyte, optionally at least a 40% or 50% augmentation in cytokine release, or optionally at least a 70% augmentation in cytokine release, and referring to the cytotoxicity assays described. In one aspect, an antibody preparation causes at least a 10% augmentation in cell surface expression of a marker of cytoxicity (e.g. CD107 and/or CD137) by a Siglec-restricted lymphocyte, optionally at least a 40% or 50% augmentation, or optionally at least a 70% augmentation in cell surface expression of a marker of cytoxicity (e.g. CD107 and/or CD137).

The ability of an anti-Siglec antibody to "block" the binding of a Siglec molecule to a sialic acid ligand means that the antibody, in an assay using soluble or cell-surface associated Siglec and sialic acid molecules, can detectably reduce the binding of a Siglec molecule to a sialic acid molecule in a dose-dependent fashion, where the Siglec molecule detectably binds to the sialic acid molecule in the absence of the antibody.

Whenever within this whole specification "treatment of cancer" or the like is mentioned with reference to anti-Siglec binding agent (e.g. antibody), there is meant: (a) method of treatment of cancer, said method comprising the step of administering (for at least one treatment) an anti-Siglec binding agent, (preferably in a pharmaceutically acceptable carrier material) to an individual, a mammal, especially a human, in need of such treatment, in a dose that allows for the treatment of cancer, (a therapeutically effective amount), preferably in a dose (amount) as specified herein; (b) the use of an anti-Siglec binding agent for the treatment of cancer, or an anti-Siglec binding agent, for use in said treatment (especially in a human); (c) the use of an anti-Siglec binding agent for the manufacture of a pharmaceutical preparation for the treatment of cancer, a method of using an anti-Siglec binding agent for the manufacture of a pharmaceutical preparation for the treatment of cancer, comprising admixing an anti-Siglec binding agent with a pharmaceutically acceptable carrier, or a pharmaceutical preparation comprising an effective dose of an anti-Siglec binding agent that is appropriate for the treatment of cancer; or (d) any combination of a), b), and c), in accordance with the subject matter allowable for patenting in a country where this application is filed.

As used herein, the term "antigen binding domain" refers to a domain comprising a three-dimensional structure capable of immunospecifically binding to an epitope. Thus, in one aspect, said domain can comprise a hypervariable region, optionally a VH and/or VL domain of an antibody chain, optionally at least a VH domain. In another aspect, the binding domain may comprise at least one complementarity determining region (CDR) of an antibody chain. In another aspect, the binding domain may comprise a polypeptide domain from a non-immunoglobulin scaffold.

The term "antibody," as used herein, refers to polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids that is primarily responsible for antigen recognition. The terms variable light chain (V_{L}) and variable heavy chain (V_{H}) refer to these light and heavy chains respectively. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG are the exemplary classes of antibodies employed herein because they are the most common antibodies in the physiological situation and because they are most easily made in a laboratory setting. Optionally the antibody is a monoclonal antibody. Particular examples of antibodies are humanized, chimeric, human, or otherwise-human-suitable antibodies. "Antibodies" also includes any fragment or derivative of any of the herein described antibodies.

A "cross-reactive" anti-Siglec antibody is an antibody that binds more than one Siglec molecule with specificity and/or affinity. For example, a monoclonal antibody can be cross-reactive with Siglec-7 and Siglec-9.

The term "specifically binds to" means that an antibody can bind preferably in a competitive binding assay to the binding partner, e.g. Siglec-7, Siglec-9, as assessed using either recombinant forms of the proteins, epitopes therein, or native proteins present on the surface of isolated target cells. Competitive binding assays and other methods for determining specific binding are further described below and are well known in the art.

When an antibody is said to "compete with" a particular monoclonal antibody, it means that the antibody competes with the monoclonal antibody in a binding assay using either recombinant Siglec molecules or surface expressed Siglec molecules. For example, if a test antibody reduces the binding of a reference antibody to a Siglec polypeptide or Siglec-expressing cell in a binding assay, the antibody is said to "compete" respectively with the reference antibody.

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Kₐ is defined by 1/Kd. Methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One standard method well known in the art for determining the affinity of mAbs is the use of surface plasmon resonance (SPR) screening (such as by analysis with a BIAcore™ SPR analytical device).

Within the context herein a "determinant" designates a site of interaction or binding on a polypeptide.

The term "epitope" refers to an antigenic determinant, and is the area or region on an antigen to which an antibody binds. A protein epitope may comprise amino acid residues directly involved in the binding as well as amino acid residues which are effectively blocked by the specific antigen binding antibody or peptide, *i.e.,* amino acid residues within the "footprint" of the antibody. It is the simplest form or smallest structural area on a complex antigen molecule that can combine with e.g., an antibody or a receptor. Epitopes can be linear or conformational/structural. The term "linear epitope" is defined as an epitope composed of amino acid residues that are contiguous on the linear sequence of amino acids (primary structure). The term "conformational or structural epitope" is defined as an epitope composed of amino acid residues that are not all contiguous and thus represent separated parts of the linear sequence of amino acids that are brought into proximity to one another by folding of the molecule (secondary, tertiary and/or quaternary structures). A conformational epitope is dependent on the 3-dimensional structure. The term 'conformational' is therefore often used interchangeably with 'structural'.

The term "deplete" or "depleting", with respect to Siglec-expressing cells (e.g. Siglec-7 or Siglec-9 expressing lymphocytes) means a process, method, or compound that results in killing, elimination, lysis or induction of such killing, elimination or lysis, so as to negatively affect the number of such Siglec-expressing cells present in a sample or in a subject. "Non-depleting", with reference to a process, method, or compound means that the process, method, or compound is not depleting.

The term "agent" is used herein to denote a chemical compound, a mixture of chemical compounds, a biological macromolecule, or an extract made from biological materials. The term "therapeutic agent" refers to an agent that has biological activity.

For the purposes herein, a "humanized" or "human" antibody refers to an antibody in which the constant and variable framework region of one or more human immunoglobulins is fused with the binding region, e.g. the CDR, of an animal immunoglobulin. Such antibodies are designed to maintain the binding specificity of the non-human antibody from which the binding regions are derived, but to avoid an immune reaction against the non-human antibody. Such antibodies can be obtained from transgenic mice or other animals that have been "engineered" to produce specific human antibodies in response to antigenic challenge (see, e.g., Green et al. (1994) Nature Genet 7:13; Lonberg et al. (1994) Nature 368:856; Taylor et al. (1994) Int Immun 6:579). A fully human antibody also can be constructed by genetic or chromosomal transfection methods, as well as phage display technology, all of which are known in the art (see, e.g., McCafferty et al. (1990) Nature 348:552-553). Human antibodies may also be generated by in vitro activated B cells (see, e.g., U.S. Pat. Nos. 5,567,610 and 5,229,275).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

The term "hypervariable region" when used herein refers to the amino acid residues of an antibody that are responsible for antigen binding. The hypervariable region generally comprises amino acid residues from a "complementarity-determining region" or "CDR" (e.g. residues 24-34 (L1), 50-56 (L2) and 89-97 (L3) in the light-chain variable domain and 31-35 (H1), 50-65 (H2) and 95-102 (H3) in the heavy-chain variable domain; Kabat et al. 1991) and/or those residues from a "hypervariable loop" (e.g. residues 26-32 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987;196:901-917), or a similar system for determining essential amino acids responsible for antigen binding. Typically, the numbering of amino acid residues in this region is performed by the method described in Kabat et al., supra. Phrases such as "Kabat position", "variable domain residue numbering as in Kabat" and "according to Kabat" herein refer to this numbering system for heavy chain variable domains or light chain variable domains. Using the Kabat numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of CDR H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

By "framework" or "FR" residues as used herein is meant the region of an antibody variable domain exclusive of those regions defined as CDRs. Each antibody variable domain framework can be further subdivided into the contiguous regions separated by the CDRs (FR1, FR2, FR3 and FR4).

The terms "Fc domain," "Fc portion," and "Fc region" refer to a C-terminal fragment of an antibody heavy chain, e.g., from about amino acid (aa) 230 to about aa 450 of human γ (gamma) heavy chain or its counterpart sequence in other types of antibody heavy chains (e.g., α, δ, ε and µ for human antibodies), or a naturally occurring allotype thereof. Unless otherwise specified, the commonly accepted Kabat amino acid numbering for immunoglobulins is used throughout this disclosure (see Kabat et al. (1991) Sequences of Protein of Immunological Interest, 5th ed., United States Public Health Service, National Institute of Health, Bethesda, MD).

The terms "isolated", "purified" or "biologically pure" refer to material that is substantially or essentially free from components which normally accompany it as found in its native state. Purity and homogeneity are typically determined using analytical chemistry techniques such as polyacrylamide gel electrophoresis or high performance liquid chromatography. A protein that is the predominant species present in a preparation is substantially purified.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under expressed or not expressed at all.

Within the context herein, the term antibody that "binds" a polypeptide or epitope designates an antibody that binds said determinant with specificity and/or affinity.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Methods for determining identity are designed to give the largest match between the sequences tested. Methods of determining identity are described in publicly available computer programs. Computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

### Production of antibodies

Within the context of this disclosure, a "common determinant" designates a determinant or epitope that is shared by several gene products of the human inhibitory Siglec receptors, notably of the CD33-related Siglecs. An antibody can bind a common determinant shared by at least Siglec-7 and Siglec-9. In one aspect, the common determinant may optionally be absent on one or more, or all of, the CD33-related Siglecs, particularly Siglecs-3, - 5, -6, -8, -10, -11 and -12. In one aspect the common determinant is absent on Siglecs-3, -5, -6, -8, -10, -11 and -12.

The anti-Siglec agent that can be used for the treatment of cancers binds an extra-cellular portion of human Siglec receptor and reduces the inhibitory activity of human Siglec receptor expressed on the surface of a Siglec positive lymphocyte. In one aspect the agent inhibits the ability of a sialic acid molecule to cause inhibitory signalling by a Siglec in a lymphocyte, e.g. an NK cell, a CD8+ T cell. In one aspect the agent competes with a sialic acid molecule in binding to a Siglec, i.e. the agent blocks the interaction between Siglec and a sialic acid ligand thereof.

In one aspect of the disclosure, the agent is an antibody selected from a full-length antibody, an antibody fragment, and a synthetic or semi-synthetic antibody-derived molecule.

In one aspect of the disclosure, the agent is an antibody selected from a fully human antibody, a humanized antibody, and a chimeric antibody.

In one aspect of the disclosure, the agent is a fragment of an antibody selected from IgA, an IgD, an IgG, an IgE and an IgM antibody.

In one aspect of the disclosure, the agent is a fragment of an antibody comprising a constant domain selected from IgG1, IgG2, IgG3 and IgG4.

In one aspect of the disclosure, the agent is an antibody fragment selected from a Fab fragment, a Fab' fragment, a Fab'-SH fragment, a F(ab)2 fragment, a F(ab')2 fragment, an Fv fragment, a Heavy chain Ig (a llama or camel Ig), a V_{HH} fragment, a single domain FV, and a single-chain antibody fragment.

In one aspect of the disclosure, the agent is a synthetic or semisynthetic antibody-derived molecule selected from a scFV, a dsFV, a minibody, a diabody, a triabody, a kappa body, an IgNAR; and a multispecific antibody.

The present disclosure thus concerns antibodies and antigen binding domains (and polypeptides comprising the foregoing) that bind to Siglec. In one aspect, the antibody or antigen binding domain binds to Siglec-7 and/or -9 with a binding affinity (e.g. KD) at least 100-fold lower than to a further human Siglec, e.g., Siglecs-3, -5, -6, -8, -10, -11 and/or -12.

In one aspect of the disclosure, the antibody is in at least partially purified form.

In one aspect of the disclosure, the antibody is in essentially isolated form.

The antibodies may be produced by a variety of techniques known in the art. Typically, they are produced by immunization of a non-human animal, preferably a mouse, with an immunogen comprising a Siglec polypeptide, preferably a human Siglec polypeptide. The Siglec polypeptide may comprise the full length sequence of a human Siglec polypeptide, or a fragment or derivative thereof, typically an immunogenic fragment, i.e., a portion of the polypeptide comprising an epitope exposed on the surface of cells expressing a Siglec polypeptide. Such fragments typically contain at least about 7 consecutive amino acids of the mature polypeptide sequence, even more preferably at least about 10 consecutive amino acids thereof. Fragments typically are essentially derived from the extra-cellular domain of the receptor. In one aspect, the immunogen comprises a wild-type human Siglec polypeptide in a lipid membrane, typically at the surface of a cell. In a specific aspect, the immunogen comprises intact cells, particularly intact human cells, optionally treated or lysed. In another aspect, the polypeptide is a recombinant Siglec polypeptide.

The step of immunizing a non-human mammal with an antigen may be carried out in any manner well known in the art for stimulating the production of antibodies in a mouse (see, for example, E. Harlow and D. Lane, Antibodies: A Laboratory Manual., Cold Spring Harbor Laboratory Press, Cold Spring Harbor, NY (1988)). The immunogen is suspended or dissolved in a buffer, optionally with an adjuvant, such as complete or incomplete Freund's adjuvant. Methods for determining the amount of immunogen, types of buffers and amounts of adjuvant are well known to those of skill in the art and are not limiting in any way. These parameters may be different for different immunogens, but are easily elucidated.

Similarly, the location and frequency of immunization sufficient to stimulate the production of antibodies is also well known in the art. In a typical immunization protocol, the non-human animals are injected intraperitoneally with antigen on day 1 and again about a week later. This is followed by recall injections of the antigen around day 20, optionally with an adjuvant such as incomplete Freund's adjuvant. The recall injections are performed intravenously and may be repeated for several consecutive days. This is followed by a booster injection at day 40, either intravenously or intraperitoneally, typically without adjuvant. This protocol results in the production of antigen-specific antibody-producing B cells after about 40 days. Other protocols may also be used as long as they result in the production of B cells expressing an antibody directed to the antigen used in immunization.

In an alternate aspect, lymphocytes from a non-immunized non-human mammal are isolated, grown in vitro, and then exposed to the immunogen in cell culture. The lymphocytes are then harvested and the fusion step described below is carried out.

For monoclonal antibodies, the next step is the isolation of splenocytes from the immunized non-human mammal and the subsequent fusion of those splenocytes with an immortalized cell in order to form an antibody-producing hybridoma. The isolation of splenocytes from a non-human mammal is well-known in the art and typically involves removing the spleen from an anesthetized non-human mammal, cutting it into small pieces and squeezing the splenocytes from the splenic capsule through a nylon mesh of a cell strainer into an appropriate buffer so as to produce a single cell suspension. The cells are washed, centrifuged and resuspended in a buffer that lyses any red blood cells. The solution is again centrifuged and remaining lymphocytes in the pellet are finally resuspended in fresh buffer.

Once isolated and present in single cell suspension, the lymphocytes can be fused to an immortal cell line. This is typically a mouse myeloma cell line, although many other immortal cell lines useful for creating hybridomas are known in the art. Murine myeloma lines include, but are not limited to, those derived from MOPC-21 and MPC-11 mouse tumors available from the Salk Institute Cell Distribution Center, San Diego, U. S. A., X63 Ag8653 and SP-2 cells available from the American Type Culture Collection, Rockville, Maryland U. S. A. The fusion is effected using polyethylene glycol or the like. The resulting hybridomas are then grown in selective media that contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Hybridomas are typically grown on a feeder layer of macrophages. The macrophages are preferably from littermates of the non-human mammal used to isolate splenocytes and are typically primed with incomplete Freund's adjuvant or the like several days before plating the hybridomas. Fusion methods are described in Goding, "Monoclonal Antibodies: Principles and Practice," pp. 59-103 (Academic Press, 1986).

The cells are allowed to grow in the selection media for sufficient time for colony formation and antibody production. This is usually between about 7 and about 14 days.

The hybridoma colonies are then assayed for the production of antibodies that specifically bind to Siglec polypeptide gene products. The assay is typically a colorimetric ELISA-type assay, although any assay may be employed that can be adapted to the wells that the hybridomas are grown in. Other assays include radioimmunoassays or fluorescence activated cell sorting. The wells positive for the desired antibody production are examined to determine if one or more distinct colonies are present. If more than one colony is present, the cells may be re-cloned and grown to ensure that only a single cell has given rise to the colony producing the desired antibody. Typically, the antibodies will also be tested for the ability to bind to Siglec polypeptides, e.g., Siglec-expressing cells.

Hybridomas that are confirmed to produce a monoclonal antibody can be grown up in larger amounts in an appropriate medium, such as DMEM or RPMI-1640. Alternatively, the hybridoma cells can be grown in vivo as ascites tumors in an animal.

After sufficient growth to produce the desired monoclonal antibody, the growth media containing monoclonal antibody (or the ascites fluid) is separated away from the cells and the monoclonal antibody present therein is purified. Purification is typically achieved by gel electrophoresis, dialysis, chromatography using protein A or protein G-Sepharose, or an anti-mouse Ig linked to a solid support such as agarose or Sepharose beads (all described, for example, in the Antibody Purification Handbook, Biosciences, publication No. 18-1037-46, Edition AC). The bound antibody is typically eluted from protein A/protein G columns by using low pH buffers (glycine or acetate buffers of pH 3.0 or less) with immediate neutralization of antibody-containing fractions. These fractions are pooled, dialyzed, and concentrated as needed.

Positive wells with a single apparent colony are typically re-cloned and re-assayed to insure only one monoclonal antibody is being detected and produced.

Antibodies may also be produced by selection of combinatorial libraries of immunoglobulins, as disclosed for instance in (Ward et al. Nature, 341 (1989) p. 544).

Once antibodies are identified that are capable of binding Siglec and/or having other desired properties, they will also typically be assessed, using standard methods including those described herein, for their ability to bind to other polypeptides, including other Siglec polypeptides and/or unrelated polypeptides. Ideally, the antibodies only bind with substantial affinity to Siglec, e.g., human Siglec-7 and human Siglec-9, and do not bind at a significant level to unrelated polypeptides, notably polypeptides other than CD33-related Siglecs, or Siglecs other than the desired Siglecs (e.g. Siglec-7 and Siglec-9). However, it will be appreciated that, as long as the affinity for Siglec is substantially greater (e.g., 5x, 10x, 50x, 100x, 500x, 1000x, 10,000x, or more) than it is for other Siglects and/or other, unrelated polypeptides), then the antibodies are suitable for use in the present methods.

Upon immunization and production of antibodies in a vertebrate or cell, particular selection steps may be performed to isolate antibodies as claimed. In this regard, the disclosure also relates to methods of producing such antibodies, comprising: (a) providing a plurality of antibodies that bind a Siglec; and (c) selecting antibodies from step (a) that are capable of binding two or more Siglecs, e.g. that bind both human Siglec-7 and human Siglec-9. In one aspect a non-human animal is used to produce the antibodies, such as a rodent, bovine, porcine, fowl, horse, rabbit, goat, or sheep. In this regard, the disclosure also relates to methods of producing such antibodies, comprising: (a) immunizing a non-human mammal with an immunogen comprising a Siglec polypeptide; and (b) preparing antibodies from said immunized animal; and (c) selecting antibodies from step (b) that are capable of binding two or more Siglecs, e.g. that bind both human Siglec-7 and human Siglec-9.

The anti-Siglec antibodies can be prepared as non-depleting antibodies such that they have reduced, or substantially lack specific binding to human Fcγ receptors. Such antibodies may comprise constant regions of various heavy chains that are known not to bind, or to have low binding affinity for, Fcγ receptors. One such example is a human IgG4 constant region. Alternatively, antibody fragments that do not comprise constant regions, such as Fab or F(ab')2 fragments, can be used to avoid Fc receptor binding. Fc receptor binding can be assessed according to methods known in the art, including for example testing binding of an antibody to Fc receptor protein in a BIACORE assay. Also, any antibody isotype can be used in which the Fc portion is modified to minimize or eliminate binding to Fc receptors (see, e.g., WO03101485). Assays such as, e.g., cell based assays, to assess Fc receptor binding are well known in the art, and are described in, e.g., WO03101485.

The DNA encoding an antibody that binds an epitope present on Siglec polypeptides is isolated from the hybridoma and placed in an appropriate expression vector for transfection into an appropriate host. The host is then used for the recombinant production of the antibody, or variants thereof, such as a humanized version of that monoclonal antibody, active fragments of the antibody, chimeric antibodies comprising the antigen recognition portion of the antibody, or versions comprising a detectable moiety.

DNA encoding a monoclonal antibodies can be readily isolated and sequenced using conventional procedures (e. g., by using oligonucleotide probes that are capable of binding specifically to genes encoding the heavy and light chains of murine antibodies). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. As described elsewhere in the present specification, such DNA sequences can be modified for any of a large number of purposes, e.g., for humanizing antibodies, producing fragments or derivatives, or for modifying the sequence of the antibody, e.g., in the antigen binding site in order to optimize the binding specificity of the antibody. Recombinant expression in bacteria of DNA encoding the antibody is well known in the art (see, for example, Skerra et al., Curr. Opinion in Immunol., 5, pp. 256 (1993); and Pluckthun, Immunol. 130, p. 151 (1992).

The identification of one or more antibodies that bind(s) to siglecs (e.g. Siglec-7 and Siglec-9, particularly substantially or essentially the same epitope as monoclonal antibody 3A11, 1H9 or 2B4, can be readily determined using any one of a variety of immunological screening assays in which antibody competition can be assessed. Many such assays are routinely practiced and are well known in the art (see, e. g., U.S. Pat. No. 5,660,827). It will be understood that actually determining the epitope to which an antibody described herein binds is not in any way required to identify an antibody that binds to the same or substantially the same epitope as the monoclonal antibody described herein.

For example, where the test antibodies to be examined are obtained from different source animals, or are even of a different Ig isotype, a simple competition assay may be employed in which the control (3A11, 1H9 or 2B4, for example) and test antibodies are admixed (or pre-adsorbed) and applied to a sample containing Siglec polypeptides. Protocols based upon western blotting and the use of BIACORE analysis are suitable for use in such competition studies.

In certain aspects, one pre-mixes the control antibodies (3A11, 1H9 or 2B4, for example) with varying amounts of the test antibodies (e.g., about 1:10 or about 1:100) for a period of time prior to applying to the Siglec antigen sample. In other aspects, the control and varying amounts of test antibodies can simply be admixed during exposure to the Siglec antigen sample. As long as one can distinguish bound from free antibodies (e. g., by using separation or washing techniques to eliminate unbound antibodies) and 3A11, 1H9 or 2B4 from the test antibodies (e. g., by using species-specific or isotype-specific secondary antibodies or by specifically labeling 3A11, 1H9 or 2B4 with a detectable label) one can determine if the test antibodies reduce the binding of 3A11, 1H9 or 2B4 to the antigens, indicating that the test antibody recognizes substantially the same epitope as 3A11, 3A11, 1H9 or 2B4. The binding of the (labeled) control antibodies in the absence of a completely irrelevant antibody can serve as the control high value. The control low value can be obtained by incubating the labeled (3A11, 1H9 or 2B4) antibodies with unlabelled antibodies of exactly the same type (3A11, 1H9 or 2B4), where competition would occur and reduce binding of the labeled antibodies. In a test assay, a significant reduction in labeled antibody reactivity in the presence of a test antibody is indicative of a test antibody that recognizes substantially the same epitope, i.e., one that "cross-reacts" or competes with the labeled (3A11, 1H9 or 2B4) antibody. Any test antibody that reduces the binding of 3A11, 1H9 or 2B4 to Siglec antigens by at least about 50%, such as at least about 60%, or more preferably at least about 80% or 90% (e. g., about 65-100%), at any ratio of 3A11, 1H9 or 2B4:test antibody between about 1:10 and about 1:100 is considered to be an antibody that binds to substantially the same epitope or determinant as 3A11, 1H9 or 2B4. Preferably, such test antibody will reduce the binding of 3A11, 1H9 or 2B4 to the Siglec antigen by at least about 90% (e.g., about 95%).

Competition can also be assessed by, for example, a flow cytometry test. In such a test, cells bearing one or more given Siglec polypeptide(s) can be incubated first with 3A11, 1H9 or 2B4, for example, and then with the test antibody labeled with a fluorochrome or biotin. The antibody is said to compete with 3A11, 1H9 or 2B4 if the binding obtained upon preincubation with a saturating amount of 3A11, 1H9 or 2B4 is about 80%, preferably about 50%, about 40% or less (e.g., about 30%, 20% or 10%) of the binding (as measured by mean of fluorescence) obtained by the antibody without preincubation with 3A11, 1H9 or 2B4. Alternatively, an antibody is said to compete with 3A11, 1H9 or 2B4 if the binding obtained with a labeled 3A11, 1H9 or 2B4 antibody (by a fluorochrome or biotin) on cells preincubated with a saturating amount of test antibody is about 80%, preferably about 50%, about 40%, or less (e. g., about 30%, 20% or 10%) of the binding obtained without preincubation with the test antibody.

A simple competition assay in which a test antibody is pre-adsorbed and applied at saturating concentration to a surface onto which a Siglec antigen is immobilized may also be employed. The surface in the simple competition assay is preferably a BIACORE chip (or other media suitable for surface plasmon resonance analysis). The control antibody (e.g., 3A11, 1H9 or 2B4) is then brought into contact with the surface at a Siglec -saturating concentration and the Siglec and surface binding of the control antibody is measured. This binding of the control antibody is compared with the binding of the control antibody to the Siglec - containing surface in the absence of test antibody. In a test assay, a significant reduction in binding of the Siglec -containing surface by the control antibody in the presence of a test antibody indicates that the test antibody recognizes substantially the same epitope as the control antibody such that the test antibody "cross-reacts" with the control antibody. Any test antibody that reduces the binding of control (such as 3A11, 1H9 or 2B4) antibody to a Siglec antigen by at least about 30% or more, preferably about 40%, can be considered to be an antibody that binds to substantially the same epitope or determinant as a control (e.g., 3A11, 1H9 or 2B4). Preferably, such a test antibody will reduce the binding of the control antibody (e.g., 3A11, 1H9 or 2B4) to the Siglec antigen by at least about 50% (e. g., at least about 60%, at least about 70%, or more). It will be appreciated that the order of control and test antibodies can be reversed: that is, the control antibody can be first bound to the surface and the test antibody is brought into contact with the surface thereafter in a competition assay. Preferably, the antibody having higher affinity for the Siglec antigen is bound to the surface first, as it will be expected that the decrease in binding seen for the second antibody (assuming the antibodies are cross-reacting) will be of greater magnitude. Further examples of such assays are provided in, e.g., Saunal (1995) J. Immunol. Methods 183: 33-41.

Determination of whether an antibody binds within an epitope region can be carried out in ways known to the person skilled in the art. As one example of such mapping/characterization methods, an epitope region for an anti-Siglec antibody may be determined by epitope "foot-printing" using chemical modification of the exposed amines/carboxyls in the Siglec protein. One specific example of such a foot-printing technique is the use of HXMS (hydrogen-deuterium exchange detected by mass spectrometry) wherein a hydrogen/deuterium exchange of receptor and ligand protein amide protons, binding, and back exchange occurs, wherein the backbone amide groups participating in protein binding are protected from back exchange and therefore will remain deuterated. Relevant regions can be identified at this point by peptic proteolysis, fast microbore high-performance liquid chromatography separation, and/or electrospray ionization mass spectrometry. See, e. g., Ehring H, Analytical Biochemistry, Vol. 267 (2) pp. 252-259 (1999) Engen, J. R. and Smith, D. L. (2001) Anal. Chem. 73, 256A-265A. Another example of a suitable epitope identification technique is nuclear magnetic resonance epitope mapping (NMR), where typically the position of the signals in two-dimensional NMR spectra of the free antigen and the antigen complexed with the antigen binding peptide, such as an antibody, are compared. The antigen typically is selectively isotopically labeled with 15N so that only signals corresponding to the antigen and no signals from the antigen binding peptide are seen in the NMR-spectrum. Antigen signals originating from amino acids involved in the interaction with the antigen binding peptide typically will shift position in the spectrum of the complex compared to the spectrum of the free antigen, and the amino acids involved in the binding can be identified that way. See, e. g., Ernst Schering Res Found Workshop. 2004; (44): 149-67; Huang et al., Journal of Molecular Biology, Vol. 281 (1) pp. 61-67 (1998); and Saito and Patterson, Methods. 1996 Jun; 9 (3): 516-24.

Epitope mapping/characterization also can be performed using mass spectrometry methods. See, e.g., Downard, J Mass Spectrom. 2000 Apr; 35 (4): 493-503 and Kiselar and Downard, Anal Chem. 1999 May 1; 71 (9): 1792-1801. Protease digestion techniques also can be useful in the context of epitope mapping and identification. Antigenic determinant-relevant regions/sequences can be determined by protease digestion, e.g. by using trypsin in a ratio of about 1:50 to Siglec or o/n digestion at and pH 7-8, followed by mass spectrometry (MS) analysis for peptide identification. The peptides protected from trypsin cleavage by the anti-Siglec binder can subsequently be identified by comparison of samples subjected to trypsin digestion and samples incubated with antibody and then subjected to digestion by e.g. trypsin (thereby revealing a footprint for the binder). Other enzymes like chymotrypsin, pepsin, etc., also or alternatively can be used in similar epitope characterization methods. Moreover, enzymatic digestion can provide a quick method for analyzing whether a potential antigenic determinant sequence is within a region of the Siglec polypeptide that is not surface exposed and, accordingly, most likely not relevant in terms of immunogenicity/antigenicity.

Site-directed mutagenesis is another technique useful for elucidation of a binding epitope. For example, in "alanine-scanning", each residue within a protein segment is replaced with an alanine residue, and the consequences for binding affinity measured. If the mutation leads to a significant reduction in binding affinity, it is most likely involved in binding. Monoclonal antibodies specific for structural epitopes (i.e., antibodies which do not bind the unfolded protein) can be used to verify that the alanine-replacement does not influence overall fold of the protein. See, e.g., Clackson and Wells, Science 1995; 267:383-386; and Wells, Proc Natl Acad Sci USA 1996; 93:1-6.

Electron microscopy can also be used for epitope "foot-printing". For example, Wang et al., Nature 1992; 355:275-278 used coordinated application of cryoelectron microscopy, three-dimensional image reconstruction, and X-ray crystallography to determine the physical footprint of a Fab-fragment on the capsid surface of native cowpea mosaic virus.

Other forms of "label-free" assay for epitope evaluation include surface plasmon resonance (SPR, BIACORE) and reflectometric interference spectroscopy (RifS). See, e.g., Fägerstam et al., Journal Of Molecular Recognition 1990;3:208-14; Nice et al., J. Chromatogr. 1993; 646:159-168; Leipert et al., Angew. Chem. Int. Ed. 1998; 37:3308-3311; Kröger et al., Biosensors and Bioelectronics 2002; 17:937-944.

It should also be noted that an antibody binding the same or substantially the same epitope as an antibody of the disclosure can be identified in one or more of the exemplary competition assays described herein.

Cross-blocking assays can also be used to evaluate whether a test (humanized) antibody affects the binding of the natural or non-natural sialic acid ligand for human Siglec (e.g. Siglec-7 and Siglec-9). For example, to determine whether a humanized anti-Siglec antibody preparation reduces or blocks Siglec-7 interactions with sialic acid, the following test can be performed: A cell line expressing sialic acid ligand for human Siglec (e.g. Siglec-7 and Siglec-9) is incubated with a labelled Siglec-Fc (e.g. Siglec-7 Fc and Siglec-9 Fc) for 1h on ice, washed, and binding analyzed on a flow cytometer by standard methods. In the absence of test antibodies, the Siglec-Fc binds to the cells. In the presence of an antibody preparation pre-incubated with Siglec-Fc (e.g. Siglec-7 Fc and Siglec-9 Fc) that blocks Siglec-binding to sialic acid, there is a reduced binding of Siglec-Fc to the cells. However, it will be appreciated that reconstitution of NK cell lytic activity toward sialic acid ligand-expressing target cells can be assessed directly without the need to assess blockade of the Siglec-sialic acid ligand interaction.

Once an antigen-binding compound having the desired binding for Siglecs is obtained it may be assessed for its ability to inhibit Siglec (e.g. Siglec-7 and/or Siglec-9). Ability to inhibit the multiple Siglecs can be assessed by testing for inhibiton of one of the Siglecs since inhibition of one Siglec will indicate the other Siglecs will also be inhibited by a cross-reactive antibody, or one may test for inhibiton of each of the Siglecs. For example, if an anti-Siglec antibody reduces or blocks Siglec activation induced by a sialic acid ligand (e.g. as present on a cell), it can increase the cytotoxicity of Siglec-restricted lymphocytes. This can be evaluated by a typical cytotoxicity assay, examples of which are described below.

The ability of an antibody to reduce Siglec-mediated signaling can be tested in a standard 4-hour in vitro cytotoxicity assay using, e.g., NK cells that express Siglec-7 or Siglec-9, and target cells that express a sialic acid ligand of the respective Siglec. Such NK cells do not efficiently kill targets that express the sialic acid ligand because Siglec-7 or -9 recognizes the sialic acid ligand, leading to initiation and propagation of inhibitory signaling that prevents lymphocyte-mediated cytolysis. Such an in vitro cytotoxicity assay can be carried out by standard methods that are well known in the art, as described for example in Coligan et al., eds., Current Protocols in Immunology, Greene Publishing Assoc. and Wiley Interscience, N.Y., (1992, 1993). The target cells are labeled with ⁵¹Cr prior to addition of NK cells, and then the killing is estimated as proportional to the release of ⁵¹Cr from the cells to the medium, as a result of killing. The addition of an antibody that prevents Siglec-7 and/or -9 from binding to the sialic acid ligand results in prevention of the initiation and propagation of inhibitory signaling via the Siglec. Therefore, addition of such agents results in increases in lymphocyte-mediated killing of the target cells. This step thereby identifies agents that prevent Siglec-7 or -9-induced negative signaling by, e.g., blocking ligand binding. In a particular ⁵¹Cr-release cytotoxicity assay, Siglec-7 or -9-expressing NK effector-cells can kill sialic acid ligand-negative target cells (e.g., cells treated with sialidase), but less well sialic acid ligand - expressing control cells. Thus, NK effector cells kill less efficiently sialic acid ligand positive cells due to sialic acid-induced inhibitory signaling via the particular Siglec. When NK cells are pre-incubated with blocking anti-Siglec antibodies in such a ⁵¹Cr-release cytotoxicity assay, sialic acid ligand-expressing cells are more efficiently killed, in an antibody-concentration-dependent fashion. The assay can be carried out separately for each Siglec, e.g. Siglec-7 and Siglec-9.

The inhibitory activity (i.e. cytotoxicity enhancing potential) of an antibody can also be assessed in any of a number of other ways, e.g., by its effect on intracellular free calcium as described, e.g., in Sivori et al., J. Exp. Med. 1997;186:1129-1136, or by the effect on markers of NK cell cytotoxicity activation, such as degranulation marker CD107 or CD137 expression. NK, T, or NKT cell activity can also be assessed using any cell based cytotoxicity assays, e.g., measuring any other parameter to assess the ability of the antibody to stimulate NK cells to kill target cells such as P815, K562 cells, or appropriate tumor cells as disclosed in Sivori et al., J. Exp. Med. 1997;186:1129-1136; Vitale et al., J. Exp. Med. 1998; 187:2065-2072; Pessino et al. J. Exp. Med. 1998;188:953-960; Neri et al. Clin. Diag. Lab. Immun. 2001;8:1131-1135; Pende et al. J. Exp. Med. 1999;190:1505-1516.

In one aspect, an antibody preparation causes at least a 10% augmentation in the cytotoxicity of a Siglec-restricted lymphocyte, preferably at least a 40% or 50% augmentation in NK cytotoxicity, or more preferably at least a 70% augmentation in NK cytotoxicity.

The activity of a cytotoxic lymphocyte can also be addressed using a cytokine-release assay, wherein NK cells are incubated with the antibody to stimulate the cytokine production of the NK cells (for example IFN-y and TNF-α production). In an exemplary protocol, IFN-y production from PBMC is assessed by cell surface and intracytoplasmic staining and analysis by flow cytometry after 4 days in culture. Briefly, Brefeldin A (Sigma Aldrich) is added at a final concentration of 5 µg/ml for the last 4 hours of culture. The cells are then incubated with anti-CD3 and anti-CD56 mAb prior to permeabilization (IntraPrep™; Beckman Coulter) and staining with PE-anti-IFN-y or PE-IgG1 (Pharmingen). GM-CSF and IFN-y production from polyclonal activated NK cells are measured in supernatants using ELISA (GM-CSF: DuoSet Elisa, R&D Systems, Minneapolis, MN, IFN-y: OptEIA set, Pharmingen).

Fragments and derivatives of antibodies (which are encompassed by the term "antibody" or "antibodies" as used in this application, unless otherwise stated or clearly contradicted by context) can be produced by techniques that are known in the art. "Fragments" comprise a portion of the intact antibody, generally the antigen binding site or variable region. Examples of antibody fragments include Fab, Fab', Fab'-SH, F (ab') 2, and Fv fragments; di-abodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific (e.g. bispecific) antibodies formed from antibody fragments. Included, *inter alia,* are a nanobody, domain antibody, single domain antibody or a "dAb".

In certain aspects, the DNA of a hybridoma producing an antibody, can be modified prior to insertion into an expression vector, for example, by substituting the coding sequence for human heavy- and light-chain constant domains in place of the homologous non-human sequences (e.g., Morrison et al., PNAS pp. 6851 (1984)), or by covalently joining to the immunoglobulin coding sequence all or part of the coding sequence for a non-immunoglobulin polypeptide. In that manner, "chimeric" or "hybrid" antibodies are prepared that have the binding specificity of the original antibody. Typically, such non-immunoglobulin polypeptides are substituted for the constant domains of an antibody.

Optionally an antibody is humanized. "Humanized" forms of antibodies are specific chimeric immunoglobulins, immunoglobulin chains or fragments thereof (such as Fv, Fab, Fab', F (ab') 2, or other antigen-binding subsequences of antibodies) which contain minimal sequence derived from the murine immunoglobulin. For the most part, humanized antibodies are human immunoglobulins (recipient antibody) in which residues from a complementary-determining region (CDR) of the recipient are replaced by residues from a CDR of the original antibody (donor antibody) while maintaining the desired specificity, affinity, and capacity of the original antibody.

In some instances, Fv framework residues of the human immunoglobulin may be replaced by corresponding non-human residues. Furthermore, humanized antibodies can comprise residues that are not found in either the recipient antibody or in the imported CDR or framework sequences. These modifications are made to further refine and optimize antibody performance. In general, the humanized antibody will comprise substantially all of at least one, and typically two, variable domains, in which all or substantially all of the CDR regions correspond to those of the original antibody and all or substantially all of the FR regions are those of a human immunoglobulin consensus sequence. The humanized antibody optimally also will comprise at least a portion of an immunoglobulin constant region (Fc), typically that of a human immunoglobulin. For further details see Jones et al., Nature, 321, pp. 522 (1986); Reichmann et al, Nature, 332, pp. 323 (1988); Presta, Curr. Op. Struct. Biol., 2, pp. 593 (1992); Verhoeyen et Science, 239, pp. 1534; and U.S. Patent No. 4,816,567); Methods for humanizing the antibodies are well known in the art.

The choice of human variable domains, both light and heavy, to be used in making the humanized antibodies is very important to reduce antigenicity. According to the so-called "best-fit" method, the sequence of the variable domain of an antibody is screened against the entire library of known human variable-domain sequences. The human sequence which is closest to that of the mouse is then accepted as the human framework (FR) for the humanized antibody (Sims et al., J. Immunol. 151, pp. 2296 (1993); Chothia and Lesk, J. Mol. 196, 1987, pp. 901). Another method uses a particular framework from the consensus sequence of all human antibodies of a particular subgroup of light or heavy chains. The same framework can be used for several different humanized antibodies (Carter et al., PNAS 89, pp. 4285 (1992); Presta et al., J. Immunol., 151, p. 2623 (1993)).

It is further important that antibodies be humanized with retention of high affinity for Siglec receptors and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequences so that the desired antibody characteristic, such as increased affinity for the target antigen (s), is achieved. In general, the CDR residues are directly and most substantially involved in influencing antigen binding.

Another method of making "humanized" monoclonal antibodies is to use a XenoMouse (Abgenix, Fremont, CA) as the mouse used for immunization. A XenoMouse is a murine host according that has had its immunoglobulin genes replaced by functional human immunoglobulin genes. Thus, antibodies produced by this mouse or in hybridomas made from the B cells of this mouse, are already humanized. The XenoMouse is described in United States Patent No. 6,162,963.

Human antibodies may also be produced according to various other techniques, such as by using, for immunization, other transgenic animals that have been engineered to express a human antibody repertoire (Jakobovitz et al., Nature 362 (1993) 255), or by selection of antibody repertoires using phage display methods. Such techniques are known to the skilled person and can be implemented starting from monoclonal antibodies as disclosed in the present application.

### Antibody CDR Sequences

### Antibody 3A11

The amino acid sequence of the heavy chain variable region of antibody 3A11 is listed as SEQ ID NO: 3, the amino acid sequence of the light chain variable region is listed as SEQ ID NO: 4. In a specific aspect, the disclosure provides an antibody that binds essentially the same epitope or determinant as monoclonal antibodies 3A11; optionally the antibody comprises the hypervariable region of antibody 3A11. In any of the aspects herein, antibody 3A11 can be characterized by the amino acid sequences and/or nucleic acid sequences encoding it. In one aspect, the monoclonal antibody comprises the Fab or F(ab')₂ portion of 3A11. Also provided is a monoclonal antibody that comprises the heavy chain variable region of 3A11. According to one aspect, the monoclonal antibody comprises the three CDRs of the heavy chain variable region of 3A11 Also provided is a monoclonal antibody that further comprises the variable light chain variable region of 3A11 or one, two or three of the CDRs of the light chain variable region of 3A11. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 3A11 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 3A11 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a HCDR2 region of 3A11 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a HCDR3 region of 3A11 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR1 region of 3A11 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR2 region of 3A11 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR3 region of 3A11 comprising an amino acid sequence as set forth in Table A, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A for each CDR), those of the Chotia numbering system as indicated in Table A for each CDR), those of the IMGT numbering system as indicated in Table A for each CDR), or any other suitable numbering system.

### Antibody 1H9

The amino acid sequence of the heavy chain variable region of antibody 1H9 is listed as SEQ ID NO: 21, the amino acid sequence of the light chain variable region is listed as SEQ ID NO: 22. In a specific aspect, the disclosure provides an antibody that binds essentially the same epitope or determinant as monoclonal antibodies 1H9; optionally the antibody comprises the hypervariable region of antibody 1H9. In any of the aspects herein, antibody 1H9 can be characterized by the amino acid sequences and/or nucleic acid sequences encoding it. In one aspect, the monoclonal antibody comprises the Fab or F(ab')₂ portion of 1H9. Also provided is a monoclonal antibody that comprises the heavy chain variable region of 1H9. According to one aspect, the monoclonal antibody comprises the three CDRs of the heavy chain variable region of 1H9 Also provided is a monoclonal antibody that further comprises the variable light chain variable region of 1H9 or one, two or three of the CDRs of the light chain variable region of 1H9. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 1H9 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 1H9 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a HCDR2 region of 1H9 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a HCDR3 region of 1H9 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR1 region of 1H9 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR2 region of 1H9 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR3 region of 1H9 comprising an amino acid sequence as set forth in Table A, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A for each CDR), those of the Chotia numbering system as indicated in Table A for each CDR), those of the IMGT numbering system as indicated in Table A for each CDR), or any other suitable numbering system.

### Antibody 2B4

The amino acid sequence of the heavy chain variable region of antibody 2B4 is listed as SEQ ID NO: 39, the amino acid sequence of the light chain variable region is listed as SEQ ID NO: 40. In a specific aspect, the disclosure provides an antibody that binds essentially the same epitope or determinant as monoclonal antibodies 2B4; optionally the antibody comprises the hypervariable region of antibody 2B4. In any of the aspects herein, antibody 2B4 can be characterized by the amino acid sequences and/or nucleic acid sequences encoding it. In one aspect, the monoclonal antibody comprises the Fab or F(ab')₂ portion of 2B4. Also provided is a monoclonal antibody that comprises the heavy chain variable region of 2B4. According to one aspect, the monoclonal antibody comprises the three CDRs of the heavy chain variable region of 2B4. Also provided is a monoclonal antibody that further comprises the variable light chain variable region of 2B4 or one, two or three of the CDRs of the light chain variable region of 2B4. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 2B4 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 2B4 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a HCDR2 region of 2B4 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a HCDR3 region of 2B4 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR1 region of 2B4 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR2 region of 2B4 comprising an amino acid sequence as set forth in Table A, or a sequence of at least 4, 5, 6, 7, 8, 9 or 10 contiguous amino acids thereof, optionally wherein one or more of these amino acids may be substituted by a different amino acid; a LCDR3 region of 2B4 comprising an amino acid sequence as set forth in Table A, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A for each CDR), those of the Chotia numbering system as indicated in Table A for each CDR), those of the IMGT numbering system as indicated in Table A for each CDR), or any other suitable numbering system.

In another aspect of any of the aspects herein, any of the CDRs 1, 2 and 3 of the heavy and light chains of 3A11, 2B4 or 1H9 may be characterized by a sequence of at least 4, 5, 6, 7, 8, 9 or 10 contiguous amino acids thereof, and/or as having an amino acid sequence that shares at least 50%, 60%, 70%, 80%, 85%, 90% or 95% sequence identity with the particular CDR or set of CDRs listed in the corresponding SEQ ID NO.

In any of the antibodies of the disclosure, e.g., 3A11, 2B4 or 1H9, the specified variable region and CDR sequences may comprise sequence modifications, e.g. a substitution (1, 2, 3, 4, 5, 6, 7, 8 or more sequence modifications). In one aspect, a CDRs 1, 2 and/or 3 of the heavy and light chains comprises one, two, three or more amino acid substitutions, where the residue substituted is a residue present in a sequence of human origin. In one aspect the substitution is a conservative modification. A conservative sequence modification refers to an amino acid modification that does not significantly affect or alter the binding characteristics of the antibody containing the amino acid sequence. Such conservative modifications include amino acid substitutions, additions and deletions. Modifications can be introduced into an antibody of the disclosure by standard techniques known in the art, such as site-directed mutagenesis and PCR-mediated mutagenesis. Conservative amino acid substitutions are typically those in which an amino acid residue is replaced with an amino acid residue having a side chain with similar physicochemical properties. Specified variable region and CDR sequences may comprise one, two, three, four or more amino acid insertions, deletions or substitutions. Where substitutions are made, preferred substitutions will be conservative modifications. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g. glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g. threonine, valine, isoleucine) and aromatic side chains (e.g., tyrosine, phenylalanine, tryptophan, histidine). Thus, one or more amino acid residues within the CDR regions of an antibody of the disclosure can be replaced with other amino acid residues from the same side chain family and the altered antibody can be tested for retained function (*i.e.,* the properties set forth herein) using the assays described herein.

The sequences of the CDRs, according to AbM (Oxford Molecular's AbM antibody modelling software definition), Kabat and Chothia definitions systems, have been summarized in Table A below. The sequences of the variable regions of the antibodies according to the disclosure are listed in Table B below (if leader sequences are present any antibody chain can be specified to start at the amino acid position immediately following the end of the leader sequence), and each CDRs underlined. In any aspect herein, a VL or VH sequence can be specified or numbered so as to contain or lack a signal peptide or any part thereof.

In one aspect, the antibodies of the disclosure are of the human IgG1 or IgG3 isotype. In one aspect, the antibodies of the disclosure are antibody fragments that retain their binding and/or functional properties.

**Table A**

| **mAb** | **CDR definition** | **HCDR1** | | **HCDR2** | | **HCDR3** | |
|---|---|---|---|---|---|---|---|
| | | **SEQ ID** | **Sequence** | **SEQ ID** | **Sequence** | **SEQ ID** | **Sequence** |
| 3A11 | IMGT | 5 | GYSFTSYW | 8 | IYPGNSDT | 11 | TTYYRYDGGFDY |
| | Chotia | 6 | GYSFTSY | 9 | PGNS | 12 | YRYDGGFD |
| | Kabat | 7 | SYWMH | 10 | AIYPGNSDTSYNQKFKD | 13 | YYRYDGGFDY |
| 1H9 | IMGT | 23 | GNTFTDYE | 26 | IHPGSGGT | 29 | TREVSGTVY |
| | Chotia | 24 | GNTFTDY | 27 | PGSG | 30 | VSGTV |
| | Kabat | 25 | DYEMH | 28 | AIHPGSGGTVYNQKFRG | 31 | EVSGTVY |
| 2B4 | IMGT | 41 | GYSFTNYW | 44 | IYPGNSDT | | TTYYRYDGGFDY |
| | Chotia | 42 | GYSFTNY | | PGNS | | YRYDGGFD |
| | Kabat | 43 | NYWMH | 45 | AIYPGNSDTSYIQKFKG | | YYRYDGGFDY |
| 3A11 | IMGT | 14 | ETVGTY | 17 | GAS | 19 | GQSYNYPYT |
| | Chotia | 15 | SETVGTY | | GAS | 20 | SYNYPY |
| | Kabat | 16 | KASETVGTYVS | 18 | GASNRNT | | GQSYNYPYT |
| 1H9 | IMGT | 32 | KSLLHSNGITY | 35 | QMS | 37 | AQNLELPWT |
| | Chotia | 33 | SKSLLHSNGITY | | QMS | 38 | NLELPW |
| | Kabat | 34 | RSSKSLLHSN-GITYLF | 36 | QMSNLAS | | AQNLELPWT |
| 2B4 | IMGT | 46 | ENVDTY | | GAS | 49 | GQSYSQPYT |
| | Chotia | 47 | SENVDTY | | GAS | 50 | SYSQPY |
| | Kabat | 48 | KASENVDTYVS | | GASNRNT | 51 | GQSYSQPYT |

**Table B**

| | **SEQ ID NO:** | **Amino Acid Sequence** |
|---|---|---|
| 3A11 VH | 3 | |
| 3A11 VL | 4 | |
| 1H9 VH | 21 | |
| 1H9 VL | 22 | |
| 2B4 VH | 39 | |
| 2B4 VL | 40 | |

### Antibody Formulations

An anti-Siglec antibody can be incorporated in a pharmaceutical formulation comprising in a concentration from 1 mg/ml to 500 mg/ml, wherein said formulation has a pH from 2.0 to 10.0. The formulation may further comprise a buffer system, preservative(s), tonicity agent(s), chelating agent(s), stabilizers and surfactants. In one aspect, the pharmaceutical formulation is an aqueous formulation, i.e., formulation comprising water. Such formulation is typically a solution or a suspension. In a further aspect, the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another aspect, the pharmaceutical formulation is a freeze-dried formulation, whereto the physician or the patient adds solvents and/or diluents prior to use.

In another aspect, the pharmaceutical formulation is a dried formulation (e.g. freeze-dried or spray-dried) ready for use without any prior dissolution.

In a further aspect, the pharmaceutical formulation comprises an aqueous solution of such an antibody, and a buffer, wherein the antibody is present in a concentration from 1 mg/ml or above, and wherein said formulation has a pH from about 2.0 to about 10.0.

In a another aspect, the pH of the formulation is in the range selected from the list consisting of from about 2.0 to about 10.0, about 3.0 to about 9.0, about 4.0 to about 8.5, about 5.0 to about 8.0, and about 5.5 to about 7.5.

In a further aspect, the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative aspect of the disclosure.

In a further aspect, the formulation further comprises a pharmaceutically acceptable preservative. In a further aspect, the formulation further comprises an isotonic agent. In a further aspect, the formulation also comprises a chelating agent. In a further aspect of the disclosure the formulation further comprises a stabilizer. In a further aspect, the formulation further comprises a surfactant.For convenience reference is made to Remington: The Science and Practice of Pharmacy, 19th edition, 1995.

It is possible that other ingredients may be present in the peptide pharmaceutical formulation of the present disclosure. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present disclosure.

Pharmaceutical compositions containing an antibody according to the present disclosure may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen. Administration of pharmaceutical compositions according to the disclosure may be through several routes of administration, for example, subcutaneous, intramuscular, intraperitoneal, intravenous, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Suitable antibody formulations can also be determined by examining experiences with other already developed therapeutic monoclonal antibodies. Several monoclonal antibodies have been shown to be efficient in clinical situations, such as Rituxan (Rituximab), Herceptin (Trastuzumab) Xolair (Omalizumab), Bexxar (Tositumomab), Campath (Alemtuzumab), Zevalin, Oncolym and similar formulations may be used with the antibodies of this disclosure. For example, a monoclonal antibody can be supplied at a concentration of 10 mg/mL in either 100 mg (10 mL) or 500 mg (50 mL) single-use vials, formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. In another aspect, the antibody is supplied in a formulation comprising about 20 mM Na-Citrate, about 150 mM NaCl, at pH of about 6.0.

### Diagnosis and treatment of malignancies

Methods of treating an individual, notably a human patient, using an anti-Siglec antibody as described herein are also provided for. In one aspect, the disclosure provides for the use of an antibody as described herein in the preparation of a pharmaceutical composition for administration to a human patient. Typically, the patient suffers from, or is at risk for, cancer or infections disease, e.g. a bacterial or a viral disease.

For example, in one aspect, the disclosure provides a method of potentiating the activity of Siglec-7 and/or -9-restricted lymphocytes in a patient in need thereof, comprising the step of administering a neutralizing anti-Siglec7/9 antibody to said patient. The antibody can be for example a human or humanized anti-Siglec-7/9 antibody, which antibody reduces or prevents sialic acid-mediated activation of the Siglec-7 and -9 receptors. In one aspect, the method directed at increasing the activity of such lymphocytes in patients having a disease in which increased lymphocyte (e.g. NK and/or CD8+ T cell) activity is beneficial, which involves, affects or is caused by cells susceptible to lysis by NK or CD8+ T cells, or which is caused or characterized by insufficient NK or CD8+ T cell activity, such as a cancer or an infectious disease.

More specifically, the methods and compositions of the present disclosure are utilized for the treatment of a variety of cancers and other proliferative diseases. Because these methods operate by enhancing an immune response via blockade of inhibitory receptors on lymphocytes, they are applicable to a very broad range of cancers. In one aspect, a human patient treated with an anti-Siglec antibody of the disclosure has liver cancer, bone cancer, pancreatic cancer, skin cancer, cancer of the head or neck, breast cancer, lung cancer, non-small cell lung cancer (NSCLC), castrate resistant prostate cancer (CRPC), melanoma, uterine cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B -lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present disclosure is also applicable to treatment of metastatic cancers. Patients can be tested or selected for one or more of the above described clinical attributes prior to, during or after treatment.

The anti-Siglec antibody based treatment can also be used to treat or prevent infectious diseases, including preferably any infections caused by infection by viruses, bacteria, protozoa, molds or fungi. Such viral infectious organisms include, but are not limited to, hepatitis type A, hepatitis type B, hepatitis type C, influenza, varicella, adenovirus, herpes simplex type I (HSV-1), herpes simplex type 2 (HSV-2), rinderpest, rhinovirus, echovirus, rota-virus, respiratory syncytial virus, papilloma virus, papilloma virus, cytomegalovirus, echinovirus, arbovirus, huntavirus, coxsackie virus, mumps virus, measles virus, rubella virus, polio virus and human immunodeficiency virus type I or type 2 (HIV-1, HIV-2). Bacteria constitute another preferred class of infectious organisms including but are not limited to the following: Staphylococcus; Streptococcus, including *S. pyogenes;* Enterococcl; Bacillus, including *Bacillus anthracis,* and Lactobacillus; Listeria; *Corynebacterium diphtheriae;* Gardnerella including *G. vaginalis;* Nocardia; Streptomyces; *Thermoactinomyces vulgaris;* Treponerna; Camplyobacter, Pseudomonas including *P. aeruginosa;* Legionella; Neisseria including *N. gonorrhoeae* and *N. meningitides;* Flavobacterium including *F. meningosepticum* and *F. odoraturn;* Brucella; Bordetella including *B. pertussis* and *B. bronchiseptica;* Escherichia including *E. coli,* Klebsiella; Enterobacter, Serratia including *S. marcescens* and *S. liquefaciens;* Edwardsiella; Proteus including *P. mirabilis* and *P. vulgaris;* Streptobacillus; Rickettsiaceae including *R. fickettsfi,* Chlamydia including *C. psittaci* and *C. trachomatis;* Mycobacterium including *M. tuberculosis, M. intracellulare, M. folluiturn, M. laprae, M. avium, M. bovis, M. africanum, M. kansasii, M. intracellulare,* and *M. lepraernurium;* and Nocardia. Protozoa may include but are not limited to, leishmania, kokzidioa, and trypanosoma. Parasites include but are not limited to, chlamydia and rickettsia.

According to another aspect, the antibody compositions may be used in combined treatments with one or more other therapeutic agents, including agents normally utilized for the particular therapeutic purpose for which the antibody is being administered. The additional therapeutic agent will normally be administered in amounts and treatment regimens typically used for that agent in a monotherapy for the particular disease or condition being treated. Such therapeutic agents include, but are not limited to anti-cancer agents, chemotherapeutic agents, antibiotics, antivirals.

In the treatment methods, the Siglec-binding compound and the second therapeutic agent can be administered separately, together or sequentially, or in a cocktail. In some aspects, the antigen-binding compound is administered prior to the administration of the second therapeutic agent. For example, the Siglec-binding compound can be administered approximately 0 to 30 days prior to the administration of the second therapeutic agent. In some aspects, a Siglec-binding compound is administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days prior to the administration of the second therapeutic agent. In some aspects, a Siglec-binding compound is administered concurrently with the administration of the therapeutic agents. In some aspects, a Siglec-binding compound is administered after the administration of the second therapeutic agent. For example, a Siglec-binding compound can be administered approximately 0 to 30 days after the administration of the second therapeutic agent. In some aspects, a Siglec-binding compound is administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days after the administration of the second therapeutic agent.

In other aspects, methods are provided for identifying Siglec-7+Siglec-9+ NK cells and/or T cells, e.g. CD8 T cells. Assessing the co-expression of Siglec-7 and Siglec-9 on NK cells and/or T cells can be used in diagnostic or prognostic methods. For example, a biological sample can be obtained from an individual (e.g. from a blood sample, from cancer or cancer-adjacent tissue obtained from a cancer patient) and analyzed for the presence of Siglec-7 and/or Siglec-9+ NK and/or T cells. The expression of both Siglec-7 and Siglec-9 on such cells can, for example, be used to identify individuals having NK and/or T cells, for example tumor infiltrating NK and/or T cells which are inhibited by both Siglec-7 and Siglec-9 polypeptides. The method can, for example, be useful as a prognostic for response to treatment with an agent that neutralizes Siglec-7 and Siglec-9.

In certain optional aspects, patients can be identified for treatment with an anti- Siglec-7/9 antibody by assessing the presence in a tumor sample (e.g. tumor tissue and/or tumor adjacent tissue) of natural ligands for Siglec-7 and/or Siglec-9. In one aspect of any of the therapeutic uses or cancer treatment or prevention methods herein, the treatment or prevention of a cancer in an individual comprises:
a) determining whether malignant cells (e.g. tumor cells) within the individual having a cancer express ligands of Siglec-7 and/or ligands of Siglec-9, and
b) upon a determination that ligands of Siglec-7 and/or ligands of Siglec-9 are significantly expressed by (e.g. on the surface of) malignant cells (e.g. tumor cells), administering to the individual an anti-Siglec7/9 antibody, e.g. an antibody according to any aspect of the disclosure.

In one aspect, a determination that a biological sample (e.g., a sample comprising tumor cells, tumor tissue and/or tumor adjacent tissue) prominently expresses ligands of Siglec-7 and/or ligands of Siglec-9 indicates that the individual has a cancer that can be treated with and/or may receive benefit from an antibody that inhibits a Siglec-7 and a Siglec-9 polypeptide.

In one aspect, significant expression of ligands of Siglec-7 and/or ligands of Siglec-9 means that said ligand(s) are expressed in a substantial number of tumor cells taken from a given individual. While not bound by a precise percentage value, in some examples a ligand can be said to be "significantly expressed" if be present on at least 30%, 40%, 50°%, 60%, 70%, 80%, or more of the tumor cells taken from a patient (in a sample).

In one aspect of any of the methods, determining whether malignant cells (e.g. tumor cells) within the individual having a cancer express ligands of Siglec-7 and/or Siglec-9 comprises determining the level of expression of ligands of Siglec-7 and/or ligands of Siglec-9 on malignant cells in a biological sample and comparing the level to a reference level (e.g. a value, weak or strong cell surface staining, etc.). The reference level may, for example, correspond to a healthy individual, to an individual deriving no/low clinical benefit from treatment with an anti-Siglec antibody, or to an individual deriving substantial clinical benefit from treatment with an anti-Siglec antibody. A determination that a biological sample expresses ligands of Siglec-7 and/or ligands of Siglec-9 at a level that is increased (e.g. a high value, strong surface staining, a level that corresponds to that of an individual deriving substantial clinical benefit from treatment with an anti-Siglec antibody, a level that is higher than that corresponding to an individual deriving no/low clinical benefit from treatment with an anti-Siglec antibody, etc.) indicates that the individual has a cancer that can be treated with an anti-Siglec antibody.

### EXAMPLES

### Example 1: A human NK cell subset that co-expresses both Siglec-7 and Siglec-9

Among the CD33-related Siglecs, Siglec-7 (CD328) and Siglec-9 (CD329) share the property of binding to sialic acids, including glycans overexpressed by cancer cells, and are thought to function as inhibitory receptors in the immune cells in which they are expressed. To investigate the expression of Siglecs on lymphocytes, distribution of Siglec-7 and Siglec-9 were studied on human NK cells.

Siglec-7 and Siglec-9 expression on NK cells was determined by flow cytometry on fresh NK cells purified from human donors. The NK population was determined as CD3-CD56+ cells (anti CD3 Pacific blue - BD Pharmingen #558124; anti CD56-PE-Vio770 - Milteny #130 100 676). Anti-Siglec-7 antibody (clone 194211 - IgG1 APC - R&D Systems #FAB11381A), anti-Siglec-9 antibody (clone 191240 - IgG2A PE - R&D Systems #FAB1139P) and isotype controls IgG1 APC and IgG2A APC were used. NK cells were incubated 30 min with 50 ul of staining Ab mix, washed twice with staining buffer, and fluorescence was revealed with Canto II (HTS).

Results are shown in Figures 1. A representative result is shown. MFI:Mean of fluorescence intensity. A significant fraction (about 44%) of NK cells expressed both Siglec-7 and Siglec-9, suggesting that a large proportion of NK cells can be inhibited by each of (or both of) these receptors, as a function of the glycan ligands present, for example on tumor cells.

### Example 2: Generation of anti-Siglec-7/9 antibodies

Siglec-7 and Siglec-9 share the characteristic domains of having a sialic acid binding N-terminal V-set Ig domain, two C2-set Ig domains and an intracytoplasmic region containing one immune-receptor tyrosine based inhibitory motif (ITIM) and one ITIM-like motif, both bind to sialic acids over-expressed by cancer cells, and are thought to have roles in tumor surveillance by way of their function as inhibitory receptors in the immune cells in which they are expressed. However, the CD33-related Siglecs are characterized, inter alia, by low evolutionary conservation and rapidly evolving sequence by multiple mechanisms. Siglec-9 shares an overall amino acid sequence identity of only about 77% with N-terminal V-set Ig domain of human Siglec-7. Moreover, these two siglecs display different sialic acids binding specificities.

Furthermore, there are a number of there are siglecs that also share sequence similarity to Siglec-7 and -9, generally divided into two groups, a first subset made up of Siglec-1, -2, -4 and -15, and the CD33-related group of Siglecs which includes Siglec-3, -5, -6, -7, -8, - 9, -10, -11, -12, -14 and -16.

To investigate whether antibodies can be obtained that bind both Siglec-7 and Siglec-9, immunizations of mice were carried out followed by screening for binding to cellular Siglec-7 and Siglec-9 (proteins expressed at the surface of cells). Additionally, since other CD33-related Siglecs have different biological functions, antibodies were further screened to assess whether it is possible to obtain cross-reactive Siglec-7/9 antibodies that do not bind to other CD33-related Siglecs.

### A. Immunization #1

To obtain anti-human Siglec-7 and Siglec-9 antibodies, Balb/c mice were immunized with a human Siglec-7 Fc and human Siglec-9 Fc extracellular domain recombinant protein. Mice received one primo-immunization with an emulsion of 30 µg of Siglec-7 Fc protein, 30 µg of human Siglec-9 Fc protein and Complete Freund Adjuvant, intraperitoneally. Mice received a second immunization with an emulsion of 30 µg of Siglec-7 Fc protein, 30 µg of human Siglec-9 Fc protein and Complete Freund Adjuvant, intraperitoneally. And finally, mice received a boost with 7.5 µg of Siglec-7 Fc protein and 7.5 µg of human Siglec-9 Fc protein, intravenously. Immune spleen cells were fused 3 days after the boost with X63.Ag8.653 immortalized B cells, and cultured in the presence of irradiated spleen cells. Hydridomas were plated in semi-solid methylcellulose-containing medium and growing clones were picked using a clonepix 2 apparatus (Molecular Devices).

Primary screen: Primary screen: Supernatant (SN) of growing clones were tested in a primary screen by flow cytometry using parental and huSiglec-7 and huSiglec-9-expressing CHO cell lines. HuSiglec-7-expressing CHO cells were stained with 0.5µM CFSE. For the flow cytometry screening, all cells were equally mixed and the presence of reacting antibodies in supernatants was revealed by Goat anti-mouse polyclonal antibody (pAb) labeled with alexa fluor 647. 20 antibodies were found to bind to human Siglec-7 and/or Siglec-9. 13 were produced as chimeric human IgG1 antibodies, and 5 of them were found to bind to both Siglec-7 and Siglec-9. Antibodies were also produced with a heavy chain N297Q (Kabat EU numbering) mutation which results in lack of N-linked glycosylation and diminished binding to Fcγ receptors, and used in further experiments.

Amino acid sequences and Genbank references for Siglec polypeptides used are shown below in Table 1.

Figure 2 shows representative results of binding to Siglec-7 and Siglec-9 for mAbs 3A11, 1H9 and 2B4, each of which bound to both human Siglec-7 and Siglec-9, as present on Siglec-7 and Siglec-9-expressing cell lines.

### B. Immunizations #2 and #3

A further series of immunization were carried out in order to assess whether additional Siglec7/9 cross-reacting antibodies can be found. The primary and secondary screens are as follows. Two additional immunizations were done following the same protocol as described in the first immunzation, except that hydridomas were plated in medium in P96 instead of semi-solid methylcellulose-containing medium. More than 30 antibodies were found to bind to both human Siglec-7 and Siglec-9.

**Table 1: Siglec sequences**

| Name | NCBI Reference Sequence | Sequence (AA) |
|---|---|---|
| Human Siglec-7 | NM_014385.3; NP_055200.1 | |
| | | |
| Human Siglec-9 | NM_014441.2;NP_055256.1 | |
| Human Siglec-3 | NM_001772.3;NP_001763.3 | |
| Human Siglec-5 | NM_003830.3 | |
| Human Siglec-6 | NM_198845.4 | |
| Human Siglec-8 | NM_014442.2 | |
| Human Siglec-10 | NM_033130.4 | |
| Human Siglec-11 | NM_052884.2 | |
| Human Siglec-12 | NM_053003.3 | |

### Example 3: Binding to CD33-related Siglecs

CD33-related Siglecs that share sequence similarity to Siglec-7 and -9 yet are generally divided into two groups, a first subset made up of Siglec-1, -2, -4 and -15, and the CD33-related group of Siglecs which includes Siglec-3, -5, -6, -7, -8, -9, -10, -11, -12, -14 and -16. Since other CD33-related Siglecs have different biological functions and/or are not thought to be involved in tumor surveillance, antibodies were further screened to assess whether it is possible to obtain cross-reactive Siglec-7/9 antibodies that do not bind to other CD33-related Siglecs.

Cells expressing Siglec-3, -5, -6, -8, -10, -11 and -12 were generated and a representative subset of the cross-reactive Siglec-7/9 antibodies were tested by flow cytometry for binding to the cells. Amino acid sequences and Genbank references for Siglec polypeptides used are shown below in Table 1.

Briefly, HuSiglec-expressing CHO cell lines (that expressed one of the Siglecs) were used. For the flow cytometry screening, antibodies were incubated 1 hour with each HuSiglec-expressing CHO cell lines (CHO HuSiglec-3 cell line, CHO HuSiglec-5 cell line, CHO HuSiglec-6 cell line, CHO HuSiglec-8 cell line, CHO HuSiglec-10 cell line, CHO HuSiglec-11 cell line, CHO HuSiglec-12 cell line), washed twice in staining buffer, revealed by Goat anti-mouse polyclonal antibody (pAb) labeled with PE, washed twice with staining buffer and stainings were acquired on a HTFC cytometer and analyzed using the FlowJo software.

Results showed that most of the cross-reactive Siglec-7/9 antibodies did not bind to any of the Siglecs, i.e. Siglec-3, -5, -6, -8, -10, -11 or -12. However, a minority of the cross-reactive Siglec-7/9 antibodies bound to Siglec-12 or Siglec-6 in addition to Siglec-7 and -9. None of the exemplary mAbs 3A11, 2B4 and 1H9 bound to any of the Siglecs-3, -5, -6, -8, - 10, -11 or-12.

### Example 4: Evaluation of ability of Siglec-7/9 to neutralize Siglec activity

Anti-Siglec-7/9 antibodies tested in the first and second immunizations were tested for blockade of Siglec activity in an NK cell activation assay. Increase of CD137 expression in 24 hours is correlated with the activation of several lymphocytes including NK cells (Kohrt et al. (2011) Blood 117(8):2423-2432). The effect of anti-Siglec-7/9 antibody and desialylation of target cells on NK cells activation was determined by analysis of CD137 expression on NK cells by flow cytometry.

Effector cells were fresh NK cells purified from donors. Target cells were Ramos cell line (wild type or neuraminidase treated) or K562 as an E:T ratio 1/1. Read out was CD137 at 24 hours. 8 donors were tested in the condition NK cells vs Ramos (Siglec-7 and Siglec-9 ligand +), in presence of anti-Siglec-7/9 antibody 3A11 (a human IgG1 isotype having an S297Q mutation as discussed above, referred to also as CHS2-M-S97A-3A11) antibody or isotype control human IgG1 having the S297Q mutation (referred to as CHS2). 4 donors were tested in the condition NK cells vs desialylated Ramos (Siglec-7 and Siglec-9 ligand -). Controls were NK cell alone, in presence of anti-Siglec-7/9 3A11 antibody or isotype control CHS2 and NK cells vs K562 as NK activation positive control cell line. Anti-Siglec-7/9 3A11 antibody whose heavy chain variable domain amino acid sequence is shown in SEQ ID NO: 3 and whose light chain variable domain amino acid sequence is shown in SEQ ID NO: 4 was used at a final concentration of 10 µg/mL. Target cells were desialylated 2 hours with 25 mU neuraminidase (Roche Diagnostics - #11080725001). Desialylation was controlled before and after neuraminidase treatment : target cells were incubated 1h in Staining Buffer (SB) with human Siglec-7 Fc (R&D Systems #1138-SL-050) and human Siglec-9 Fc recombinant protein (R&D Systems #1139-SL-050) at 10ug/ml, washed twice with SB, incubated 30 min with a Goat F(ab')2 Anti-Mouse IgG (Fc) PE (Beckman Coulter #IM0551), washed twice with SB, and fluorescence was revealed with Canto II (HTS). The CD137 assay was carried out in 96 U well plates in completed RPMI, 200µL final/well. Antibodies, target cells and effector cells were mixed; spin 1 min at 300g; incubated 24h at 37°C; spin 3 min at 500g; washed twice with Staining Buffer (SB); added 50µL of staining Ab mix (anti CD3 Pacific blue - BD Pharmingen #558124; anti CD56-PE-Vio770 - Miltenyi #130 100 676; anti CD137-APC - Miltenyi #130 094 821) ; incubated 30 min at 300g; washed twice with SB resuspended pellet with SB ; and fluorescence revealed with Canto II (HTS).

Figures 3 shows CD137 FACS read-outs on NK cells in presence of target cells or desialylated target cells and in presence of 3A11 anti-Siglec-7/9 antibody or isotype control CHS2 antibody at a saturating dose of 10 µg/mL. Each dot represents NK from a healthy volunteer. 3A11 antibody induced an increase of CD137 expression on NK cells in presence of the target cell line Ramos. 3A11 antibody had no effect on CD137 expression on NK cells in absence of target cell line. Similarly, 3A11 antibody induced an increase of lysis of Ramos target cell line in a cytotoxicity assay. Induction of CD137 expression on NK cells was also observed with desialylated Ramos target cells with 2 donors (D2 and D3). For two other donors (D1 and D4), 3A11 induced an increase of CD137 expression but not desialylated target cells, suggesting that 3A11 blocks the interaction between Siglec-7 and Siglec-9 and sialic acid ligands but also sialic acid-free ligands, i.e. other unknown ligands (independently of sialic acid). Three additional antibodies (one cross reactive anti Siglec-7/9 and two anti Siglec-9 antibodies) induced also a weak increase of CD137 expression in a CD137 assay. From these results, we can conclude that the 3A11 blocks the Siglec-7/-9 inhibitory signal and increase NK activation and cytotoxicity.

## Claims

1. An isolated monoclonal antibody that specifically binds to a human Siglec-7 polypeptide and to a human Siglec-9 polypeptide, wherein the antibody causes an increase in CD137 expression in Siglec-expressing NK cells when the NK cells are brought into contact with a target human cell bearing a ligand of the Siglec on the target cell surface.

2. The antibody of claim 1, wherein the antibody lacks an Fc domain, is a human IgG4 isotype antibody, or is an antibody having an Fc domain that is modified to reduce binding between the Fc domain and a human Fcy receptor.

3. The antibody of claims 1-2, which does not substantially bind to a third human CD33-related Siglec polypeptide, wherein the third human CD33-related Siglec is selected from the group consisting of Siglec-3, -5, -6, -8, -10, -11 and -12.

4. The isolated antibody of claim 3, wherein the third human CD33-related Siglec polypeptide is Siglec-12.

5. The antibody of claims 1-4, which inhibits activation of the Siglec by a natural a ligand the Siglec present on a cell.

6. The monoclonal antibody of claims 1-5 **characterized by**:
a) specifically binding to Siglec-7, and when bound to Siglec-7 on a human immune cell, increasing activation and/or cytotoxicity of said immune cell toward a target human cell bearing a ligand of Siglec-7 on the target cell surface, when said target cell comes into contact with said immune cell;
b) specifically binding to Siglec-9, and when bound to Siglec-9 on a human lymphocyte, increasing activation and/or cytotoxicity of said immune cell toward a target human cell bearing a ligand of Siglec-9 on the target cell surface, when said target cell comes into contact with said immune cell; and
c) not substantially binding to the CD16 human Fcy receptor.

7. The antibody of any one of the above claims, wherein said ligand of Siglec-7 and/or Siglec-9 comprises a sialic acid.

8. The antibody of any one of the above claims, wherein the antibody comprises (i) a heavy chain variable region comprising CDR 1, 2 and 3 of the heavy chain variable region of SEQ ID NO: 3 and (ii) a light chain variable region comprising CDR 1, 2 and 3 of the light chain variable region of SEQ ID NO: 4.

9. The antibody of any one of the above claims, wherein the antibody comprises two antigen binding domains capable of binding to Siglec-7 and to Siglec-9.

10. A pharmaceutical composition comprising an antibody according to any one of the above claims, and a pharmaceutically acceptable carrier.

11. A nucleic acid encoding a heavy and light chain of an antibody of claim 8.

12. A hybridoma or recombinant host cell producing the antibody of any one of claims 1 to 9.

13. An antibody of any one of claims 1-9 or a composition of claim 10, for the treatment or prevention of cancer.

14. An antibody or composition for use of claim 13, wherein the antibody or composition is administered to a subject in an effective amount to modulate CD56^{dim} NK cells and CD56^{bright} NK cells.

15. A method of producing a monoclonal antibody which cross-reacts with multiple Siglec gene products and which neutralizes the inhibitory activity of such Siglecs, said method comprising the steps of:
(a) providing a plurality of monoclonal antibodies that bind Siglec-7 and Siglec-9 polypeptides,
(b) selecting antibodies that cross-react with Siglec-7 and Siglec-9 polypeptides, and
(c) selecting antibodies that neutralize the inhibitory activity of Siglec-7 and Siglec-9 polypeptides.

## Patentansprüche

1. Ein isolierter monoklonaler Antikörper, der an ein humanes Siglec-7-Polypeptid und an ein humanes Siglec-9-Polypeptid spezifisch bindet, wobei der Antikörper einen Anstieg der CD137-Expression in Siglec-exprimierenden NK-Zellen hervorruft, wenn die NK-Zellen in Kontakt mit einer humanen Zielzelle gebracht werden, die einen Liganden des Siglec auf der Zielzellenoberfläche trägt.

2. Der Antikörper nach Anspruch 1, wobei der Antikörper ohne eine Fc-Domäne ist, ein humaner IgG4-Isotyp-Antikörper ist oder ein Antikörper mit einer Fc-Domäne ist, die modifiziert ist, um ein Binden zwischen der Fc-Domäne und einem humanen Fcy-Rezeptor zu verringern.

3. Der Antikörper nach Ansprüchen 1 bis 2, der nicht wesentlich an ein drittes humanes CD33-verwandtes Siglec-Polypeptid bindet, wobei das dritte CD33-verwandte Siglec aus der Gruppe ausgewählt ist, bestehend aus Siglec-3, -5, -6, -8, -10, -11 und -12.

4. Der isolierte Antikörper nach Anspruch 3, wobei das dritte humane CD33-verwandte Siglec-Polypeptid Siglec-12 ist.

5. Der isolierte Antikörper nach Ansprüchen 1 bis 4, der eine Aktivierung des Siglec durch einen auf einer Zelle vorhandenen natürlichen Liganden des Siglec hemmt.

6. Der monoklonale Antikörper nach Ansprüchen 1 bis 5, **gekennzeichnet durch**:
a) spezifisches Binden an Siglec-7, und wenn er an Siglec-7 auf einer humanen Immunzelle gebunden ist, die Aktivierung und/oder Zytotoxizität besagter Immunzelle gegen eine humane Zielzelle erhöht, die einen Liganden von Siglec-7 auf der Zielzellenoberfläche trägt, wenn besagte Zielzelle in Kontakt mit besagter Immunzelle kommt;
b) spezifisches Binden an Siglec-9, und wenn er an Siglec-9 auf einem humanen Lymphozyten gebunden ist, die Aktivierung und/oder Zytotoxizität besagter Immunzelle gegen eine humane Zielzelle erhöht, die einen Liganden von Siglec-9 auf der Zielzellenoberfläche trägt, wenn besagte Zielzelle in Kontakt mit besagter Immunzelle kommt; und
c) im Wesentlichen nicht an den CD16 humanen Fcy-Rezeptor bindet.

7. Der Antikörper nach einem der obigen Ansprüche, wobei besagter Ligand von Siglec-7 und/oder Siglec-9 eine Sialsäure umfasst.

8. Der Antikörper nach einem der obigen Ansprüche, wobei der Antikörper umfasst (i) eine variable Region der schweren Kette, umfassend CDR 1, 2 und 3 der variablen Region der schweren Kette von SEQ ID NO: 3 und (ii) eine variable Region der leichten Kette, umfassend CDR 1, 2 und 3 der variablen Region der leichten Kette von SEQ ID NO: 4.

9. Der Antikörper nach einem der obigen Ansprüche, wobei der Antikörper zwei antigenbindende Domänen umfasst, die in der Lage sind, an Siglec-7 und an Siglec-9 zu binden.

10. Eine pharmazeutische Zusammensetzung, umfassend einen Antikörper gemäß einem der obigen Ansprüche und einen pharmazeutisch verträglichen Träger.

11. Eine Nukleinsäure, codierend eine schwere und leichte Kette eines Antikörpers nach Anspruch 8.

12. Ein Hybridom oder eine rekombinante Wirtszelle, produzierend den Antikörper nach einem der Ansprüche 1 bis 9.

13. Ein Antikörper nach einem der Ansprüche 1 bis 9 oder eine Zusammensetzung nach Anspruch 10 für die Behandlung oder Prävention von Krebs.

14. Ein Antikörper oder eine Zusammensetzung zur Verwendung nach Anspruch 13, wobei der Antikörper oder die Zusammensetzung an eine Person in einer wirksamen Menge verabreicht wird, um CD56^{dim} NK-Zellen und CD56^{bright} NK-Zellen zu modulieren.

15. Ein Verfahren zur Herstellung eines monoklonalen Antikörpers, der mit mehreren Siglec-Genprodukten kreuzreagiert und der die Hemmaktivität derartiger Siglecs neutralisiert, wobei besagtes Verfahren die Schritte umfasst:
(a) Bereitstellen einer Vielzahl an monoklonalen Antikörpern, die Siglec-7- und Siglec-9-Polypeptide binden,
(b) Selektieren von Antikörpern, die mit Siglec-7- und Siglec-9-Polypeptiden kreuzreagieren, und
(c) Selektieren von Antikörpern, die die Hemmaktivität von Siglec-7- und Siglec-9-Polypeptiden neutralisieren.

## Revendications

1. Anticorps monoclonal isolé qui se lie spécifiquement à un polypeptide Siglec-7 humain et à un polypeptide Siglec-9 humain, lequel anticorps provoque une augmentation de l'expression de CD137 dans des cellules NK exprimant un Siglec quand les cellules NK sont mises en contact avec une cellule humaine cible portant un ligand du Siglec sur la surface de la cellule cible.

2. Anticorps selon la revendication 1, lequel anticorps est dépourvu d'un domaine Fc, est un anticorps d'isotype IgG4 humain, ou est un anticorps ayant un domaine Fc qui est modifié pour réduire la liaison entre le domaine Fc et un récepteur Fcy humain.

3. Anticorps selon les revendications 1 et 2, qui ne se lie pas sensiblement à un troisième polypeptide Siglec apparenté au CD33 humain, dans lequel le troisième Siglec apparenté au CD33 humain est choisi dans le groupe constitué par les Siglec-3, -5, -6, -8, -10, -11 et -12.

4. Anticorps isolé selon la revendication 3, dans lequel le troisième polypeptide Siglec apparenté au CD33 humain est le Siglec-12.

5. Anticorps selon les revendications 1 à 4, qui inhibe l'activation du Siglec par un ligand naturel du Siglec présent sur une cellule.

6. Anticorps monoclonal selon les revendications 1 à 5, **caractérisé en ce que** :
a) il se lie spécifiquement au Siglec-7, et quand il est lié au Siglec-7 sur une cellule immunitaire humaine, il augmente l'activation et/ou la cytotoxicité de ladite cellule immunitaire vis-à-vis d'une cellule humaine cible portant un ligand de Siglec-7 sur la surface de la cellule cible, quand ladite cellule cible vient en contact avec ladite cellule immunitaire ;
b) il se lie spécifiquement au Siglec-9, et quand il est lié au Siglec-9 sur un lymphocyte humain, il augmente l'activation et/ou la cytotoxicité de ladite cellule immunitaire vis-à-vis d'une cellule humaine cible portant un ligand de Siglec-9 sur la surface de la cellule cible, quand ladite cellule cible vient en contact avec ladite cellule immunitaire ; et
c) il ne se lie pas sensiblement au récepteur Fcy humain CD16.

7. Anticorps selon l'une quelconque des revendications précédentes, dans lequel ledit ligand de Siglec-7 et/ou Siglec-9 comprend un acide sialique.

8. Anticorps selon l'une quelconque des revendications précédentes, dans lequel l'anticorps comprend (i) une région variable de chaîne lourde comprenant les CDR 1, 2 et 3 de la région variable de chaîne lourde de la SEQ ID NO : 3 et (ii) une région variable de chaîne légère comprenant les CDR 1, 2 et 3 de la région variable de chaîne légère de la SEQ ID NO : 4.

9. Anticorps selon l'une quelconque des revendications précédentes, lequel anticorps comprend deux domaines de liaison à un antigène capable de se lier au Siglec-7 et/ou au Siglec-9.

10. Composition pharmaceutique comprenant un anticorps selon l'une quelconque des revendications précédentes, et un véhicule pharmaceutiquement acceptable.

11. Acide nucléique codant une chaîne lourde et une chaîne légère d'un anticorps de la revendication 8.

12. Hybridome ou cellule hôte recombinée produisant l'anticorps de l'une quelconque des revendications 1 à 9.

13. Anticorps selon l'une quelconque des revendications 1 à 9 ou composition selon la revendication 10, pour le traitement ou la prévention d'un cancer.

14. Anticorps ou composition pour une utilisation selon la revendication 13, lequel anticorps ou composition est administré à un sujet en une quantité efficace pour moduler des cellules NK CD56^{dim} et des cellules NK CD56^{bright}.

15. Méthode de production d'un anticorps monoclonal qui réagit de façon croisée avec de multiples produits géniques de Siglec et qui neutralise l'activité inhibitrice de ces Siglec, ladite méthode comprenant les étapes suivantes :
(a) obtention d'une pluralité d'anticorps monoclonaux qui se lient à des polypeptides Siglec-7 et Siglec-9,
(b) sélection d'anticorps qui réagissent de façon croisée avec les polypeptides Siglec-7 et Siglec-9, et
(c) sélection d'anticorps qui neutralisent l'activité inhibitrice des polypeptides Siglec-7 et Siglec-9.


### SEQUENCE LISTING

<110> INNATE PHARMA
<120> CROSS REACTIVE SIGLEC ANTIBODIES
<130> Sig7-9
<150> US 62/048,292
   <151> 2014-09-10
<160> 60
<170> PatentIn version 3.5
<210> 1
   <211> 467
   <212> PRT
   <213> Homo sapiens
<400> 1
   Met Leu Leu Leu Leu Leu Leu Pro Leu Leu Trp Gly Arg Glu Arg Val
   1               5                   10                  15
   Glu Gly Gln Lys Ser Asn Arg Lys Asp Tyr Ser Leu Thr Met Gln Ser
               20                  25                  30
   Ser Val Thr Val Gln Glu Gly Met Cys Val His Val Arg Cys Ser Phe
           35                  40                  45
   Ser Tyr Pro Val Asp Ser Gln Thr Asp Ser Asp Pro Val His Gly Tyr
       50                  55                  60
   Trp Phe Arg Ala Gly Asn Asp Ile Ser Trp Lys Ala Pro Val Ala Thr
   65                  70                  75                  80
   Asn Asn Pro Ala Trp Ala Val Gln Glu Glu Thr Arg Asp Arg Phe His
                   85                  90                  95
   Leu Leu Gly Asp Pro Gln Thr Lys Asn Cys Thr Leu Ser Ile Arg Asp
               100                 105                 110
   Ala Arg Met Ser Asp Ala Gly Arg Tyr Phe Phe Arg Met Glu Lys Gly
           115                 120                 125
   Asn Ile Lys Trp Asn Tyr Lys Tyr Asp Gln Leu Ser Val Asn Val Thr
       130                 135                 140
   Ala Leu Thr His Arg Pro Asn Ile Leu Ile Pro Gly Thr Leu Glu Ser
   145                 150                 155                 160
   Gly Cys Phe Gln Asn Leu Thr Cys Ser Val Pro Trp Ala Cys Glu Gln
   165 170 175
   Gly Thr Pro Pro Met Ile Ser Trp Met Gly Thr Ser Val Ser Pro Leu
   180 185 190
   His Pro Ser Thr Thr Arg Ser Ser Val Leu Thr Leu Ile Pro Gln Pro
   195 200 205
   Gln His His Gly Thr Ser Leu Thr Cys Gln Val Thr Leu Pro Gly Ala
   210 215 220
   Gly Val Thr Thr Asn Arg Thr Ile Gln Leu Asn Val Ser Tyr Pro Pro
   225 230 235 240
   Gln Asn Leu Thr Val Thr Val Phe Gln Gly Glu Gly Thr Ala Ser Thr
   245 250 255
   Ala Leu Gly Asn Ser Ser Ser Leu Ser Val Leu Glu Gly Gln Ser Leu
   260 265 270
   Arg Leu Val Cys Ala Val Asp Ser Asn Pro Pro Ala Arg Leu Ser Trp
   275 280 285
   Thr Trp Arg Ser Leu Thr Leu Tyr Pro Ser Gln Pro Ser Asn Pro Leu
   290 295 300
   Val Leu Glu Leu Gln Val His Leu Gly Asp Glu Gly Glu Phe Thr Cys
   305 310 315 320
   Arg Ala Gln Asn Ser Leu Gly Ser Gln His Val Ser Leu Asn Leu Ser
   325 330 335
   Leu Gln Gln Glu Tyr Thr Gly Lys Met Arg Pro Val Ser Gly Val Leu
   340 345 350
   Leu Gly Ala Val Gly Gly Ala Gly Ala Thr Ala Leu Val Phe Leu Ser
   355 360 365
   Phe Cys Val Ile Phe Ile Val Val Arg Ser Cys Arg Lys Lys Ser Ala
   370 375 380
   Arg Pro Ala Ala Asp Val Gly Asp Ile Gly Met Lys Asp Ala Asn Thr
   385 390 395 400
   Ile Arg Gly Ser Ala Ser Gln Gly Asn Leu Thr Glu Ser Trp Ala Asp
   405 410 415
   Asp Asn Pro Arg His His Gly Leu Ala Ala His Ser Ser Gly Glu Glu
               420                 425                 430
   Arg Glu Ile Gln Tyr Ala Pro Leu Ser Phe His Lys Gly Glu Pro Gln
           435                 440                 445
   Asp Leu Ser Gly Gln Glu Ala Thr Asn Asn Glu Tyr Ser Glu Ile Lys
       450                 455                 460
   Ile Pro Lys
   465
<210> 2
   <211> 463
   <212> PRT
   <213> Homo sapiens
<400> 2
   Met Leu Leu Leu Leu Leu Pro Leu Leu Trp Gly Arg Glu Arg Ala Glu
   1               5                   10                  15
   Gly Gln Thr Ser Lys Leu Leu Thr Met Gln Ser Ser Val Thr Val Gln
               20                  25                  30
   Glu Gly Leu Cys Val His Val Pro Cys Ser Phe Ser Tyr Pro Ser His
           35                  40                  45
   Gly Trp Ile Tyr Pro Gly Pro Val Val His Gly Tyr Trp Phe Arg Glu
       50                  55                  60
   Gly Ala Asn Thr Asp Gln Asp Ala Pro Val Ala Thr Asn Asn Pro Ala
   65                  70                  75                  80
   Arg Ala Val Trp Glu Glu Thr Arg Asp Arg Phe His Leu Leu Gly Asp
                   85                  90                  95
   Pro His Thr Lys Asn Cys Thr Leu Ser Ile Arg Asp Ala Arg Arg Ser
               100                 105                 110
   Asp Ala Gly Arg Tyr Phe Phe Arg Met Glu Lys Gly Ser Ile Lys Trp
           115                 120                 125
   Asn Tyr Lys His His Arg Leu Ser Val Asn Val Thr Ala Leu Thr His
       130                 135                 140
   Arg Pro Asn Ile Leu Ile Pro Gly Thr Leu Glu Ser Gly Cys Pro Gln
   145                 150                 155                 160
   Asn Leu Thr Cys Ser Val Pro Trp Ala Cys Glu Gln Gly Thr Pro Pro
                   165                 170                 175
   Met Ile Ser Trp Ile Gly Thr Ser Val Ser Pro Leu Asp Pro Ser Thr
               180                 185                 190
   Thr Arg Ser Ser Val Leu Thr Leu Ile Pro Gln Pro Gln Asp His Gly
           195                 200                 205
   Thr Ser Leu Thr Cys Gln Val Thr Phe Pro Gly Ala Ser Val Thr Thr
       210                 215                 220
   Asn Lys Thr Val His Leu Asn Val Ser Tyr Pro Pro Gln Asn Leu Thr
   225                 230                 235                 240
   Met Thr Val Phe Gln Gly Asp Gly Thr Val Ser Thr Val Leu Gly Asn
                   245                 250                 255
   Gly Ser Ser Leu Ser Leu Pro Glu Gly Gln Ser Leu Arg Leu Val Cys
               260                 265                 270
   Ala Val Asp Ala Val Asp Ser Asn Pro Pro Ala Arg Leu Ser Leu Ser
           275                 280                 285
   Trp Arg Gly Leu Thr Leu Cys Pro Ser Gln Pro Ser Asn Pro Gly Val
       290                 295                 300
   Leu Glu Leu Pro Trp Val His Leu Arg Asp Ala Ala Glu Phe Thr Cys
   305                 310                 315                 320
   Arg Ala Gln Asn Pro Leu Gly Ser Gln Gln Val Tyr Leu Asn Val Ser
                   325                 330                 335
   Leu Gln Ser Lys Ala Thr Ser Gly Val Thr Gln Gly Val Val Gly Gly
               340                 345                 350
   Ala Gly Ala Thr Ala Leu Val Phe Leu Ser Phe Cys Val Ile Phe Val
           355                 360                 365
   Val Val Arg Ser Cys Arg Lys Lys Ser Ala Arg Pro Ala Ala Gly Val
       370                 375                 380
   Gly Asp Thr Gly Ile Glu Asp Ala Asn Ala Val Arg Gly Ser Ala Ser
   385                 390                 395                 400
   Gln Gly Pro Leu Thr Glu Pro Trp Ala Glu Asp Ser Pro Pro Asp Gln
                   405                 410                 415
   Pro Pro Pro Ala Ser Ala Arg Ser Ser Val Gly Glu Gly Glu Leu Gln
   420 425 430
   Tyr Ala Ser Leu Ser Phe Gln Met Val Lys Pro Trp Asp Ser Arg Gly
   435 440 445
   Gln Glu Ala Thr Asp Thr Glu Tyr Ser Glu Ile Lys Ile His Arg
   450 455 460
<210> 3
   <211> 119
   <212> PRT
   <213> Mus musculus
<400> 3
   Glu Val Gln Leu Gln Gln Ser Gly Thr Val Leu Ala Arg Pro Gly Ala
   1 5 10 15
   Ser Val Lys Met Ser Cys Lys Ala Ser Gly Tyr Ser Phe Thr Ser Tyr
   20 25 30
   Trp Met His Trp Val Lys Gln Arg Pro Gly Gln Gly Leu Glu Trp Ile
   35 40 45
   Gly Ala Ile Tyr Pro Gly Asn Ser Asp Thr Ser Tyr Asn Gln Lys Phe
   50 55 60
   Lys Asp Lys Ala Lys Leu Thr Ala Val Thr Ser Ala Ser Thr Ala Tyr
   65 70 75 80
   Met Glu Leu Ser Ser Leu Thr Asn Glu Asp Ser Ala Val Tyr Tyr Cys
   85 90 95
   Thr Thr Tyr Tyr Arg Tyr Asp Gly Gly Phe Asp Tyr Trp Gly Gln Gly
   100 105 110
   Thr Thr Leu Thr Val Ser Ser
   115
<210> 4
   <211> 107
   <212> PRT
   <213> Mus musculus
<400> 4
   Asp Ile Gln Met Thr Gln Ser Pro Lys Ser Met Ser Met Ser Val Gly
   1 5 10 15
   Glu Arg Val Thr Leu Ser Cys Lys Ala Ser Glu Thr Val Gly Thr Tyr
   20 25 30
   Val Ser Trp Tyr Gln Gln Lys Pro Glu Gln Ser Pro Lys Leu Leu Ile
   35 40 45
   Phe Gly Ala Ser Asn Arg Asn Thr Gly Val Pro Asp Arg Phe Thr Gly
   50 55 60
   Ser Gly Ser Ala Thr Asp Phe Thr Leu Thr Ile Ser Ser Val Gln Ala
   65 70 75 80
   Glu Asp Leu Ala Asp Tyr His Cys Gly Gln Ser Tyr Asn Tyr Pro Tyr
   85 90 95
   Thr Phe Gly Gly Gly Thr Lys Leu Glu Ile Lys
   100 105
<210> 5
   <211> 8
   <212> PRT
   <213> Mus musculus
<400> 5
   Gly Tyr Ser Phe Thr Ser Tyr Trp
   1 5
<210> 6
   <211> 7
   <212> PRT
   <213> Mus musculus
<400> 6
   Gly Tyr Ser Phe Thr Ser Tyr
   1 5
<210> 7
   <211> 5
   <212> PRT
   <213> Mus musculus
<400> 7
   Ser Tyr Trp Met His
   1 5
<210> 8
   <211> 8
   <212> PRT
   <213> Mus musculus
<400> 8
   Ile Tyr Pro Gly Asn Ser Asp Thr
   1 5
<210> 9
   <211> 4
   <212> PRT
   <213> Mus musculus
<400> 9
   Pro Gly Asn Ser
   1
<210> 10
   <211> 17
   <212> PRT
   <213> Mus musculus
<400> 10
   Ala Ile Tyr Pro Gly Asn Ser Asp Thr Ser Tyr Asn Gln Lys Phe Lys
   1 5 10 15
   Asp
<210> 11
   <211> 12
   <212> PRT
   <213> Mus musculus
<400> 11
   Thr Thr Tyr Tyr Arg Tyr Asp Gly Gly Phe Asp Tyr
   1 5 10
<210> 12
   <211> 8
   <212> PRT
   <213> Mus musculus
<400> 12
   Tyr Arg Tyr Asp Gly Gly Phe Asp
   1 5
<210> 13
   <211> 10
   <212> PRT
   <213> Mus musculus
<400> 13
   Tyr Tyr Arg Tyr Asp Gly Gly Phe Asp Tyr
   1 5 10
<210> 14
   <211> 6
   <212> PRT
   <213> Mus musculus
<400> 14
   Glu Thr Val Gly Thr Tyr
   1 5
<210> 15
   <211> 7
   <212> PRT
   <213> Mus musculus
<400> 15
   Ser Glu Thr Val Gly Thr Tyr
   1 5
<210> 16
   <211> 11
   <212> PRT
   <213> Mus musculus
<400> 16
   Lys Ala Ser Glu Thr Val Gly Thr Tyr Val Ser
   1 5 10
<210> 17
   <211> 3
   <212> PRT
   <213> Mus musculus
<400> 17
   Gly Ala Ser
   1
<210> 18
   <211> 7
   <212> PRT
   <213> Mus musculus
<400> 18
   Gly Ala Ser Asn Arg Asn Thr
   1 5
<210> 19
   <211> 9
   <212> PRT
   <213> Mus musculus
<400> 19
   Gly Gln Ser Tyr Asn Tyr Pro Tyr Thr
   1 5
<210> 20
   <211> 6
   <212> PRT
   <213> Mus musculus
<400> 20
   Ser Tyr Asn Tyr Pro Tyr
   1 5
<210> 21
   <211> 116
   <212> PRT
   <213> Mus musculus
<400> 21
   Gln Val Gln Leu Gln Gln Ser Gly Ala Glu Leu Val Arg Pro Gly Ala
   1 5 10 15
   Ser Val Lys Leu Ser Cys Lys Ala Met Gly Asn Thr Phe Thr Asp Tyr
   20 25 30
   Glu Met His Trp Val Lys Gln Thr Pro Val His Gly Leu Glu Trp Ile
   35 40 45
   Gly Ala Ile His Pro Gly Ser Gly Gly Thr Val Tyr Asn Gln Lys Phe
   50 55 60
   Arg Gly Lys Ala Ala Leu Thr Ala Asp Lys Ser Ser Ser Thr Ala Tyr
   65 70 75 80
   Met Glu Leu Ser Ser Leu Thr Ser Glu Asp Ser Ala Val Tyr Tyr Cys
   85 90 95
   Thr Arg Glu Val Ser Gly Thr Val Tyr Trp Gly Gln Gly Thr Thr Leu
   100 105 110
   Thr Val Ser Ser
   115
<210> 22
   <211> 112
   <212> PRT
   <213> Mus musculus
<400> 22
   Asp Ile Val Met Thr Gln Ala Ala Phe Ser Asn Pro Val Thr Leu Gly
   1 5 10 15
   Thr Ser Ala Ser Ile Ser Cys Arg Ser Ser Lys Ser Leu Leu His Ser
   20 25 30
   Asn Gly Ile Thr Tyr Leu Phe Trp Tyr Leu Gln Lys Pro Gly Gln Ser
   35 40 45
   Pro Gln Leu Leu Ile Tyr Gln Met Ser Asn Leu Ala Ser Gly Val Pro
   50 55 60
   Asp Arg Phe Ser Ser Ser Gly Ser Gly Thr Asp Phe Thr Leu Arg Ile
   65 70 75 80
   Ser Arg Val Glu Ala Glu Asp Val Gly Val Tyr Tyr Cys Ala Gln Asn
   85 90 95
   Leu Glu Leu Pro Trp Thr Phe Gly Gly Gly Thr Lys Leu Glu Ile Lys
   100 105 110
<210> 23
   <211> 8
   <212> PRT
   <213> Mus musculus
<400> 23
   Gly Asn Thr Phe Thr Asp Tyr Glu
   1 5
<210> 24
   <211> 7
   <212> PRT
   <213> Mus musculus
<400> 24
   Gly Asn Thr Phe Thr Asp Tyr
   1 5
<210> 25
   <211> 5
   <212> PRT
   <213> Mus musculus
<400> 25
   Asp Tyr Glu Met His
   1 5
<210> 26
   <211> 8
   <212> PRT
   <213> Mus musculus
<400> 26
   Ile His Pro Gly Ser Gly Gly Thr
   1 5
<210> 27
   <211> 4
   <212> PRT
   <213> Mus musculus
<400> 27
   Pro Gly Ser Gly
   1
<210> 28
   <211> 17
   <212> PRT
   <213> Mus musculus
<400> 28
   Ala Ile His Pro Gly Ser Gly Gly Thr Val Tyr Asn Gln Lys Phe Arg
   1 5 10 15
   Gly
<210> 29
   <211> 9
   <212> PRT
   <213> Mus musculus
<400> 29
   Thr Arg Glu Val Ser Gly Thr Val Tyr
   1 5
<210> 30
   <211> 5
   <212> PRT
   <213> Mus musculus
<400> 30
   Val Ser Gly Thr Val
   1 5
<210> 31
   <211> 7
   <212> PRT
   <213> Mus musculus
<400> 31
   Glu Val Ser Gly Thr Val Tyr
   1 5
<210> 32
   <211> 11
   <212> PRT
   <213> Mus musculus
<400> 32
   Lys Ser Leu Leu His Ser Asn Gly Ile Thr Tyr
   1               5                   10
<210> 33
   <211> 12
   <212> PRT
   <213> Mus musculus
<400> 33
   Ser Lys Ser Leu Leu His Ser Asn Gly Ile Thr Tyr
   1               5                   10
<210> 34
   <211> 16
   <212> PRT
   <213> Mus musculus
<400> 34
   Arg Ser Ser Lys Ser Leu Leu His Ser Asn Gly Ile Thr Tyr Leu Phe
   1               5                   10                  15
<210> 35
   <211> 3
   <212> PRT
   <213> Mus musculus
<400> 35
   Gln Met Ser
   1
<210> 36
   <211> 7
   <212> PRT
   <213> Mus musculus
<400> 36
   Gln Met Ser Asn Leu Ala Ser
   1               5
<210> 37
   <211> 9
   <212> PRT
   <213> Mus musculus
<400> 37
   Ala Gln Asn Leu Glu Leu Pro Trp Thr
   1 5
<210> 38
   <211> 6
   <212> PRT
   <213> Mus musculus
<400> 38
   Asn Leu Glu Leu Pro Trp
   1 5
<210> 39
   <211> 119
   <212> PRT
   <213> Mus musculus
<400> 39
   Glu Val Gln Leu Gln Gln Ser Gly Thr Val Leu Ala Arg Pro Gly Ala
   1 5 10 15
   Ser Val Lys Met Ser Cys Lys Ala Ser Gly Tyr Ser Phe Thr Asn Tyr
   20 25 30
   Trp Met His Trp Val Lys Gln Arg Pro Gly Gln Gly Leu Glu Trp Ile
   35 40 45
   Gly Ala Ile Tyr Pro Gly Asn Ser Asp Thr Ser Tyr Ile Gln Lys Phe
   50 55 60
   Lys Gly Lys Ala Lys Leu Thr Ala Val Thr Ser Ala Ser Thr Ala Tyr
   65 70 75 80
   Met Glu Leu Ser Ser Leu Thr Asn Glu Asp Ser Ala Val Tyr Tyr Cys
   85 90 95
   Thr Thr Tyr Tyr Arg Tyr Asp Gly Gly Phe Asp Tyr Trp Gly Gln Gly
   100 105 110
   Thr Thr Leu Thr Val Ser Ser
   115
<210> 40
   <211> 107
   <212> PRT
   <213> Mus musculus
<400> 40
   Asp Ile Val Met Thr Gln Ser Pro Lys Ser Met Ser Met Ser Val Gly
   1 5 10 15
   Glu Arg Val Thr Leu Ser Cys Lys Ala Ser Glu Asn Val Asp Thr Tyr
   20 25 30
   Val Ser Trp Tyr Gln Gln Lys Pro Asp Gln Ser Pro Lys Leu Leu Leu
   35 40 45
   Tyr Gly Ala Ser Asn Arg Asn Thr Gly Val Pro Asp Arg Phe Thr Gly
   50 55 60
   Ser Gly Ser Ala Thr Asp Phe Thr Leu Thr Ile Ser Asn Val Gln Ala
   65 70 75 80
   Glu Asp Leu Ala Asp Tyr His Cys Gly Gln Ser Tyr Ser Gln Pro Tyr
   85 90 95
   Thr Phe Gly Gly Gly Thr Lys Leu Glu Ile Lys
   100 105
<210> 41
   <211> 8
   <212> PRT
   <213> Mus musculus
<400> 41
   Gly Tyr Ser Phe Thr Asn Tyr Trp
   1 5
<210> 42
   <211> 7
   <212> PRT
   <213> Mus musculus
<400> 42
   Gly Tyr Ser Phe Thr Asn Tyr
   1 5
<210> 43
   <211> 5
   <212> PRT
   <213> Mus musculus
<400> 43
   Asn Tyr Trp Met His
   1 5
<210> 44
   <211> 8
   <212> PRT
   <213> Mus musculus
<400> 44
   Ile Tyr Pro Gly Asn Ser Asp Thr
   1               5
<210> 45
   <211> 17
   <212> PRT
   <213> Mus musculus
<400> 45
   Ala Ile Tyr Pro Gly Asn Ser Asp Thr Ser Tyr Ile Gln Lys Phe Lys
   1               5                   10                  15
   Gly
<210> 46
   <211> 6
   <212> PRT
   <213> Mus musculus
<400> 46
   Glu Asn Val Asp Thr Tyr
   1               5
<210> 47
   <211> 7
   <212> PRT
   <213> Mus musculus
<400> 47
   Ser Glu Asn Val Asp Thr Tyr
   1               5
<210> 48
   <211> 11
   <212> PRT
   <213> Mus musculus
<400> 48
   Lys Ala Ser Glu Asn Val Asp Thr Tyr Val Ser
   1               5                   10
<210> 49
   <211> 9
   <212> PRT
   <213> Mus musculus
<400> 49
   Gly Gln Ser Tyr Ser Gln Pro Tyr Thr
   1 5
<210> 50
   <211> 6
   <212> PRT
   <213> Mus musculus
<400> 50
   Ser Tyr Ser Gln Pro Tyr
   1 5
<210> 51
   <211> 9
   <212> PRT
   <213> Mus musculus
<400> 51
   Gly Gln Ser Tyr Ser Gln Pro Tyr Thr
   1 5
<210> 52
   <211> 449
   <212> PRT
   <213> Homo sapiens
<400> 52
   Gln Lys Ser Asn Arg Lys Asp Tyr Ser Leu Thr Met Gln Ser Ser Val
   1 5 10 15
   Thr Val Gln Glu Gly Met Cys Val His Val Arg Cys Ser Phe Ser Tyr
   20 25 30
   Pro Val Asp Ser Gln Thr Asp Ser Asp Pro Val His Gly Tyr Trp Phe
   35 40 45
   Arg Ala Gly Asn Asp Ile Ser Trp Lys Ala Pro Val Ala Thr Asn Asn
   50 55 60
   Pro Ala Trp Ala Val Gln Glu Glu Thr Arg Asp Arg Phe His Leu Leu
   65 70 75 80
   Gly Asp Pro Gln Thr Lys Asn Cys Thr Leu Ser Ile Arg Asp Ala Arg
   85 90 95
   Met Ser Asp Ala Gly Arg Tyr Phe Phe Arg Met Glu Lys Gly Asn Ile
   100 105 110
   Lys Trp Asn Tyr Lys Tyr Asp Gln Leu Ser Val Asn Val Thr Ala Leu
               115                 120                 125
   Thr His Arg Pro Asn Ile Leu Ile Pro Gly Thr Leu Glu Ser Gly Cys
       130                 135                 140
   Phe Gln Asn Leu Thr Cys Ser Val Pro Trp Ala Cys Glu Gln Gly Thr
   145                 150                 155                 160
   Pro Pro Met Ile Ser Trp Met Gly Thr Ser Val Ser Pro Leu His Pro
                   165                 170                 175
   Ser Thr Thr Arg Ser Ser Val Leu Thr Leu Ile Pro Gln Pro Gln His
               180                 185                 190
   His Gly Thr Ser Leu Thr Cys Gln Val Thr Leu Pro Gly Ala Gly Val
           195                 200                 205
   Thr Thr Asn Arg Thr Ile Gln Leu Asn Val Ser Tyr Pro Pro Gln Asn
       210                 215                 220
   Leu Thr Val Thr Val Phe Gln Gly Glu Gly Thr Ala Ser Thr Ala Leu
   225                 230                 235                 240
   Gly Asn Ser Ser Ser Leu Ser Val Leu Glu Gly Gln Ser Leu Arg Leu
                   245                 250                 255
   Val Cys Ala Val Asp Ser Asn Pro Pro Ala Arg Leu Ser Trp Thr Trp
               260                 265                 270
   Arg Ser Leu Thr Leu Tyr Pro Ser Gln Pro Ser Asn Pro Leu Val Leu
           275                 280                 285
   Glu Leu Gln Val His Leu Gly Asp Glu Gly Glu Phe Thr Cys Arg Ala
       290                 295                 300
   Gln Asn Ser Leu Gly Ser Gln His Val Ser Leu Asn Leu Ser Leu Gln
   305                 310                 315                 320
   Gln Glu Tyr Thr Gly Lys Met Arg Pro Val Ser Gly Val Leu Leu Gly
                   325                 330                 335
   Ala Val Gly Gly Ala Gly Ala Thr Ala Leu Val Phe Leu Ser Phe Cys
               340                 345                 350
   Val Ile Phe Ile Val Val Arg Ser Cys Arg Lys Lys Ser Ala Arg Pro
           355                 360                 365
   Ala Ala Asp Val Gly Asp Ile Gly Met Lys Asp Ala Asn Thr Ile Arg
   370 375 380
   Gly Ser Ala Ser Gln Gly Asn Leu Thr Glu Ser Trp Ala Asp Asp Asn
   385 390 395 400
   Pro Arg His His Gly Leu Ala Ala His Ser Ser Gly Glu Glu Arg Glu
   405 410 415
   Ile Gln Tyr Ala Pro Leu Ser Phe His Lys Gly Glu Pro Gln Asp Leu
   420 425 430
   Ser Gly Gln Glu Ala Thr Asn Asn Glu Tyr Ser Glu Ile Lys Ile Pro
   435 440 445
   Lys
<210> 53
   <211> 446
   <212> PRT
   <213> Homo sapiens
<400> 53
   Gln Thr Ser Lys Leu Leu Thr Met Gln Ser Ser Val Thr Val Gln Glu
   1 5 10 15
   Gly Leu Cys Val His Val Pro Cys Ser Phe Ser Tyr Pro Ser His Gly
   20 25 30
   Trp Ile Tyr Pro Gly Pro Val Val His Gly Tyr Trp Phe Arg Glu Gly
   35 40 45
   Ala Asn Thr Asp Gln Asp Ala Pro Val Ala Thr Asn Asn Pro Ala Arg
   50 55 60
   Ala Val Trp Glu Glu Thr Arg Asp Arg Phe His Leu Leu Gly Asp Pro
   65 70 75 80
   His Thr Lys Asn Cys Thr Leu Ser Ile Arg Asp Ala Arg Arg Ser Asp
   85 90 95
   Ala Gly Arg Tyr Phe Phe Arg Met Glu Lys Gly Ser Ile Lys Trp Asn
   100 105 110
   Tyr Lys His His Arg Leu Ser Val Asn Val Thr Ala Leu Thr His Arg
   115 120 125
   Pro Asn Ile Leu Ile Pro Gly Thr Leu Glu Ser Gly Cys Pro Gln Asn
       130                 135                 140
   Leu Thr Cys Ser Val Pro Trp Ala Cys Glu Gln Gly Thr Pro Pro Met
   145                 150                 155                 160
   Ile Ser Trp Ile Gly Thr Ser Val Ser Pro Leu Asp Pro Ser Thr Thr
                   165                 170                 175
   Arg Ser Ser Val Leu Thr Leu Ile Pro Gln Pro Gln Asp His Gly Thr
               180                 185                 190
   Ser Leu Thr Cys Gln Val Thr Phe Pro Gly Ala Ser Val Thr Thr Asn
           195                 200                 205
   Lys Thr Val His Leu Asn Val Ser Tyr Pro Pro Gln Asn Leu Thr Met
       210                 215                 220
   Thr Val Phe Gln Gly Asp Gly Thr Val Ser Thr Val Leu Gly Asn Gly
   225                 230                 235                 240
   Ser Ser Leu Ser Leu Pro Glu Gly Gln Ser Leu Arg Leu Val Cys Ala
                   245                 250                 255
   Val Asp Ala Val Asp Ser Asn Pro Pro Ala Arg Leu Ser Leu Ser Trp
               260                 265                 270
   Arg Gly Leu Thr Leu Cys Pro Ser Gln Pro Ser Asn Pro Gly Val Leu
           275                 280                 285
   Glu Leu Pro Trp Val His Leu Arg Asp Ala Ala Glu Phe Thr Cys Arg
       290                 295                 300
   Ala Gln Asn Pro Leu Gly Ser Gln Gln Val Tyr Leu Asn Val Ser Leu
   305                 310                 315                 320
   Gln Ser Lys Ala Thr Ser Gly Val Thr Gln Gly Val Val Gly Gly Ala
                   325                 330                 335
   Gly Ala Thr Ala Leu Val Phe Leu Ser Phe Cys Val Ile Phe Val Val
               340                 345                 350
   Val Arg Ser Cys Arg Lys Lys Ser Ala Arg Pro Ala Ala Gly Val Gly
           355                 360                 365
   Asp Thr Gly Ile Glu Asp Ala Asn Ala Val Arg Gly Ser Ala Ser Gln
       370                 375                 380
   Gly Pro Leu Thr Glu Pro Trp Ala Glu Asp Ser Pro Pro Asp Gln Pro
   385                 390                 395                 400
   Pro Pro Ala Ser Ala Arg Ser Ser Val Gly Glu Gly Glu Leu Gln Tyr
                   405                 410                 415
   Ala Ser Leu Ser Phe Gln Met Val Lys Pro Trp Asp Ser Arg Gly Gln
               420                 425                 430
   Glu Ala Thr Asp Thr Glu Tyr Ser Glu Ile Lys Ile His Arg
           435                 440                 445
<210> 54
   <211> 347
   <212> PRT
   <213> Homo sapiens
<400> 54
   Asp Pro Asn Phe Trp Leu Gln Val Gln Glu Ser Val Thr Val Gln Glu
   1               5                   10                  15
   Gly Leu Cys Val Leu Val Pro Cys Thr Phe Phe His Pro Ile Pro Tyr
               20                  25                  30
   Tyr Asp Lys Asn Ser Pro Val His Gly Tyr Trp Phe Arg Glu Gly Ala
           35                  40                  45
   Ile Ile Ser Gly Asp Ser Pro Val Ala Thr Asn Lys Leu Asp Gln Glu
       50                  55                  60
   Val Gln Glu Glu Thr Gln Gly Arg Phe Arg Leu Leu Gly Asp Pro Ser
   65                  70                  75                  80
   Arg Asn Asn Cys Ser Leu Ser Ile Val Asp Ala Arg Arg Arg Asp Asn
                   85                  90                  95
   Gly Ser Tyr Phe Phe Arg Met Glu Arg Gly Ser Thr Lys Tyr Ser Tyr
               100                 105                 110
   Lys Ser Pro Gln Leu Ser Val His Val Thr Asp Leu Thr His Arg Pro
           115                 120                 125
   Lys Ile Leu Ile Pro Gly Thr Leu Glu Pro Gly His Ser Lys Asn Leu
       130                 135                 140
   Thr Cys Ser Val Ser Trp Ala Cys Glu Gln Gly Thr Pro Pro Ile Phe
   145                 150                 155                 160
   Ser Trp Leu Ser Ala Ala Pro Thr Ser Leu Gly Pro Arg Thr Thr His
                   165                 170                 175
   Ser Ser Val Leu Ile Ile Thr Pro Arg Pro Gln Asp His Gly Thr Asn
               180                 185                 190
   Leu Thr Cys Gln Val Lys Phe Ala Gly Ala Gly Val Thr Thr Glu Arg
           195                 200                 205
   Thr Ile Gln Leu Asn Val Thr Tyr Val Pro Gln Asn Pro Thr Thr Gly
       210                 215                 220
   Ile Phe Pro Gly Asp Gly Ser Gly Lys Gln Glu Thr Arg Ala Gly Val
   225                 230                 235                 240
   Val His Gly Ala Ile Gly Gly Ala Gly Val Thr Ala Leu Leu Ala Leu
                   245                 250                 255
   Cys Leu Cys Leu Ile Phe Phe Ile Val Lys Thr His Arg Arg Lys Ala
               260                 265                 270
   Ala Arg Thr Ala Val Gly Arg Asn Asp Thr His Pro Thr Thr Gly Ser
           275                 280                 285
   Ala Ser Pro Lys His Gln Lys Lys Ser Lys Leu His Gly Pro Thr Glu
       290                 295                 300
   Thr Ser Ser Cys Ser Gly Ala Ala Pro Thr Val Glu Met Asp Glu Glu
   305                 310                 315                 320
   Leu His Tyr Ala Ser Leu Asn Phe His Gly Met Asn Pro Ser Lys Asp
                   325                 330                 335
   Thr Ser Thr Glu Tyr Ser Glu Val Arg Thr Gln
               340                 345
<210> 55
   <211> 535
   <212> PRT
   <213> Homo sapiens
<400> 55
   Glu Lys Pro Val Tyr Glu Leu Gln Val Gln Lys Ser Val Thr Val Gln
   1               5                   10                  15
   Glu Gly Leu Cys Val Leu Val Pro Cys Ser Phe Ser Tyr Pro Trp Arg
               20                  25                  30
   Ser Trp Tyr Ser Ser Pro Pro Leu Tyr Val Tyr Trp Phe Arg Asp Gly
           35                  40                  45
   Glu Ile Pro Tyr Tyr Ala Glu Val Val Ala Thr Asn Asn Pro Asp Arg
       50                  55                  60
   Arg Val Lys Pro Glu Thr Gln Gly Arg Phe Arg Leu Leu Gly Asp Val
   65                  70                  75                  80
   Gln Lys Lys Asn Cys Ser Leu Ser Ile Gly Asp Ala Arg Met Glu Asp
                   85                  90                  95
   Thr Gly Ser Tyr Phe Phe Arg Val Glu Arg Gly Arg Asp Val Lys Tyr
               100                 105                 110
   Ser Tyr Gln Gln Asn Lys Leu Asn Leu Glu Val Thr Ala Leu Ile Glu
           115                 120                 125
   Lys Pro Asp Ile His Phe Leu Glu Pro Leu Glu Ser Gly Arg Pro Thr
       130                 135                 140
   Arg Leu Ser Cys Ser Leu Pro Gly Ser Cys Glu Ala Gly Pro Pro Leu
   145                 150                 155                 160
   Thr Phe Ser Trp Thr Gly Asn Ala Leu Ser Pro Leu Asp Pro Glu Thr
                   165                 170                 175
   Thr Arg Ser Ser Glu Leu Thr Leu Thr Pro Arg Pro Glu Asp His Gly
               180                 185                 190
   Thr Asn Leu Thr Cys Gln Met Lys Arg Gln Gly Ala Gln Val Thr Thr
           195                 200                 205
   Glu Arg Thr Val Gln Leu Asn Val Ser Tyr Ala Pro Gln Thr Ile Thr
       210                 215                 220
   Ile Phe Arg Asn Gly Ile Ala Leu Glu Ile Leu Gln Asn Thr Ser Tyr
   225                 230                 235                 240
   Leu Pro Val Leu Glu Gly Gln Ala Leu Arg Leu Leu Cys Asp Ala Pro
                   245                 250                 255
   Ser Asn Pro Pro Ala His Leu Ser Trp Phe Gln Gly Ser Pro Ala Leu
               260                 265                 270
   Asn Ala Thr Pro Ile Ser Asn Thr Gly Ile Leu Glu Leu Arg Arg Val
           275                 280                 285
   Arg Ser Ala Glu Glu Gly Gly Phe Thr Cys Arg Ala Gln His Pro Leu
   290 295 300
   Gly Phe Leu Gln Ile Phe Leu Asn Leu Ser Val Tyr Ser Leu Pro Gln
   305 310 315 320
   Leu Leu Gly Pro Ser Cys Ser Trp Glu Ala Glu Gly Leu His Cys Arg
   325 330 335
   Cys Ser Phe Arg Ala Arg Pro Ala Pro Ser Leu Cys Trp Arg Leu Glu
   340 345 350
   Glu Lys Pro Leu Glu Gly Asn Ser Ser Gln Gly Ser Phe Lys Val Asn
   355 360 365
   Ser Ser Ser Ala Gly Pro Trp Ala Asn Ser Ser Leu Ile Leu His Gly
   370 375 380
   Gly Leu Ser Ser Asp Leu Lys Val Ser Cys Lys Ala Trp Asn Ile Tyr
   385 390 395 400
   Gly Ser Gln Ser Gly Ser Val Leu Leu Leu Gln Gly Arg Ser Asn Leu
   405 410 415
   Gly Thr Gly Val Val Pro Ala Ala Leu Gly Gly Ala Gly Val Met Ala
   420 425 430
   Leu Leu Cys Ile Cys Leu Cys Leu Ile Phe Phe Leu Ile Val Lys Ala
   435 440 445
   Arg Arg Lys Gln Ala Ala Gly Arg Pro Glu Lys Met Asp Asp Glu Asp
   450 455 460
   Pro Ile Met Gly Thr Ile Thr Ser Gly Ser Arg Lys Lys Pro Trp Pro
   465 470 475 480
   Asp Ser Pro Gly Asp Gln Ala Ser Pro Pro Gly Asp Ala Pro Pro Leu
   485 490 495
   Glu Glu Gln Lys Glu Leu His Tyr Ala Ser Leu Ser Phe Ser Glu Met
   500 505 510
   Lys Ser Arg Glu Pro Lys Asp Gln Glu Ala Pro Ser Thr Thr Glu Tyr
   515 520 525
   Ser Glu Ile Lys Thr Ser Lys
   530 535
<210> 56
   <211> 411
   <212> PRT
   <213> Homo sapiens
<400> 56
   Gln Glu Arg Arg Phe Gln Leu Glu Gly Pro Glu Ser Leu Thr Val Gln
   1               5                   10                  15
   Glu Gly Leu Cys Val Leu Val Pro Cys Arg Leu Pro Thr Thr Leu Pro
               20                  25                  30
   Ala Ser Tyr Tyr Gly Tyr Gly Tyr Trp Phe Leu Glu Gly Ala Asp Val
           35                  40                  45
   Pro Val Ala Thr Asn Asp Pro Asp Glu Glu Val Gln Glu Glu Thr Arg
       50                  55                  60
   Gly Arg Phe His Leu Leu Trp Asp Pro Arg Arg Lys Asn Cys Ser Leu
   65                  70                  75                  80
   Ser Ile Arg Asp Ala Arg Arg Arg Asp Asn Ala Ala Tyr Phe Phe Arg
                   85                  90                  95
   Leu Lys Ser Lys Trp Met Lys Tyr Gly Tyr Thr Ser Ser Lys Leu Ser
               100                 105                 110
   Val Arg Val Met Ala Leu Thr His Arg Pro Asn Ile Ser Ile Pro Gly
           115                 120                 125
   Thr Leu Glu Ser Gly His Pro Ser Asn Leu Thr Cys Ser Val Pro Trp
       130                 135                 140
   Val Cys Glu Gln Gly Thr Pro Pro Ile Phe Ser Trp Met Ser Ala Ala
   145                 150                 155                 160
   Pro Thr Ser Leu Gly Pro Arg Thr Thr Gln Ser Ser Val Leu Thr Ile
                   165                 170                 175
   Thr Pro Arg Pro Gln Asp His Ser Thr Asn Leu Thr Cys Gln Val Thr
               180                 185                 190
   Phe Pro Gly Ala Gly Val Thr Met Glu Arg Thr Ile Gln Leu Asn Val
           195                 200                 205
   Ser Ser Phe Lys Ile Leu Gln Asn Thr Ser Ser Leu Pro Val Leu Glu
           210                 215                 220
   Gly Gln Ala Leu Arg Leu Leu Cys Asp Ala Asp Gly Asn Pro Pro Ala
   225                 230                 235                 240
   His Leu Ser Trp Phe Gln Gly Phe Pro Ala Leu Asn Ala Thr Pro Ile
                   245                 250                 255
   Ser Asn Thr Gly Val Leu Glu Leu Pro Gln Val Gly Ser Ala Glu Glu
               260                 265                 270
   Gly Asp Phe Thr Cys Arg Ala Gln His Pro Leu Gly Ser Leu Gln Ile
           275                 280                 285
   Ser Leu Ser Leu Phe Val His Trp Lys Pro Glu Gly Arg Ala Gly Gly
       290                 295                 300
   Val Leu Gly Ala Val Trp Gly Ala Ser Ile Thr Thr Leu Val Phe Leu
   305                 310                 315                 320
   Cys Val Cys Phe Ile Phe Arg Val Lys Thr Arg Arg Lys Lys Ala Ala
                   325                 330                 335
   Gln Pro Val Gln Asn Thr Asp Asp Val Asn Pro Val Met Val Ser Gly
               340                 345                 350
   Ser Arg Gly His Gln His Gln Phe Gln Thr Gly Ile Val Ser Asp His
           355                 360                 365
   Pro Ala Glu Ala Gly Pro Ile Ser Glu Asp Glu Gln Glu Leu His Tyr
       370                 375                 380
   Ala Val Leu His Phe His Lys Val Gln Pro Gln Glu Pro Lys Val Thr
   385                 390                 395                 400
   Asp Thr Glu Tyr Ser Glu Ile Lys Ile His Lys
                   405                 410
<210> 57
   <211> 483
   <212> PRT
   <213> Homo sapiens
<400> 57
   Met Glu Gly Asp Arg Gln Tyr Gly Asp Gly Tyr Leu Leu Gln Val Gln
   1               5                   10                  15
   Glu Leu Val Thr Val Gln Glu Gly Leu Cys Val His Val Pro Cys Ser
   20 25 30
   Phe Ser Tyr Pro Gln Asp Gly Trp Thr Asp Ser Asp Pro Val His Gly
   35 40 45
   Tyr Trp Phe Arg Ala Gly Asp Arg Pro Tyr Gln Asp Ala Pro Val Ala
   50 55 60
   Thr Asn Asn Pro Asp Arg Glu Val Gln Ala Glu Thr Gln Gly Arg Phe
   65 70 75 80
   Gln Leu Leu Gly Asp Ile Trp Ser Asn Asp Cys Ser Leu Ser Ile Arg
   85 90 95
   Asp Ala Arg Lys Arg Asp Lys Gly Ser Tyr Phe Phe Arg Leu Glu Arg
   100 105 110
   Gly Ser Met Lys Trp Ser Tyr Lys Ser Gln Leu Asn Tyr Lys Thr Lys
   115 120 125
   Gln Leu Ser Val Phe Val Thr Ala Leu Thr His Arg Pro Asp Ile Leu
   130 135 140
   Ile Leu Gly Thr Leu Glu Ser Gly His Ser Arg Asn Leu Thr Cys Ser
   145 150 155 160
   Val Pro Trp Ala Cys Lys Gln Gly Thr Pro Pro Met Ile Ser Trp Ile
   165 170 175
   Gly Ala Ser Val Ser Ser Pro Gly Pro Thr Thr Ala Arg Ser Ser Val
   180 185 190
   Leu Thr Leu Thr Pro Lys Pro Gln Asp His Gly Thr Ser Leu Thr Cys
   195 200 205
   Gln Val Thr Leu Pro Gly Thr Gly Val Thr Thr Thr Ser Thr Val Arg
   210 215 220
   Leu Asp Val Ser Tyr Pro Pro Trp Asn Leu Thr Met Thr Val Phe Gln
   225 230 235 240
   Gly Asp Ala Thr Ala Ser Thr Ala Leu Gly Asn Gly Ser Ser Leu Ser
   245 250 255
   Val Leu Glu Gly Gln Ser Leu Arg Leu Val Cys Ala Val Asn Ser Asn
   260 265 270
   Pro Pro Ala Arg Leu Ser Trp Thr Arg Gly Ser Leu Thr Leu Cys Pro
   275 280 285
   Ser Arg Ser Ser Asn Pro Gly Leu Leu Glu Leu Pro Arg Val His Val
   290 295 300
   Arg Asp Glu Gly Glu Phe Thr Cys Arg Ala Gln Asn Ala Gln Gly Ser
   305 310 315 320
   Gln His Ile Ser Leu Ser Leu Ser Leu Gln Asn Glu Gly Thr Gly Thr
   325 330 335
   Ser Arg Pro Val Ser Gln Val Thr Leu Ala Ala Val Gly Gly Ala Gly
   340 345 350
   Ala Thr Ala Leu Ala Phe Leu Ser Phe Cys Ile Ile Phe Ile Ile Val
   355 360 365
   Arg Ser Cys Arg Lys Lys Ser Ala Arg Pro Ala Ala Gly Val Gly Asp
   370 375 380
   Thr Gly Met Glu Asp Ala Lys Ala Ile Arg Gly Ser Ala Ser Gln Gly
   385 390 395 400
   Pro Leu Thr Glu Ser Trp Lys Asp Gly Asn Pro Leu Lys Lys Pro Pro
   405 410 415
   Pro Ala Val Ala Pro Ser Ser Gly Glu Glu Gly Glu Leu His Tyr Ala
   420 425 430
   Thr Leu Ser Phe His Lys Val Lys Pro Gln Asp Pro Gln Gly Gln Glu
   435 440 445
   Ala Thr Asp Ser Glu Tyr Ser Glu Ile Lys Ile His Lys Arg Glu Thr
   450 455 460
   Ala Glu Thr Gln Ala Cys Leu Arg Asn His Asn Pro Ser Ser Lys Glu
   465 470 475 480
   Val Arg Gly
<210> 58
   <211> 681
   <212> PRT
   <213> Homo sapiens
<400> 58
   Met Asp Gly Arg Phe Trp Ile Arg Val Gln Glu Ser Val Met Val Pro
   1               5                   10                  15
   Glu Gly Leu Cys Ile Ser Val Pro Cys Ser Phe Ser Tyr Pro Arg Gln
               20                  25                  30
   Asp Trp Thr Gly Ser Thr Pro Ala Tyr Gly Tyr Trp Phe Lys Ala Val
           35                  40                  45
   Thr Glu Thr Thr Lys Gly Ala Pro Val Ala Thr Asn His Gln Ser Arg
       50                  55                  60
   Glu Val Glu Met Ser Thr Arg Gly Arg Phe Gln Leu Thr Gly Asp Pro
   65                  70                  75                  80
   Ala Lys Gly Asn Cys Ser Leu Val Ile Arg Asp Ala Gln Met Gln Asp
                   85                  90                  95
   Glu Ser Gln Tyr Phe Phe Arg Val Glu Arg Gly Ser Tyr Val Arg Tyr
               100                 105                 110
   Asn Phe Met Asn Asp Gly Phe Phe Leu Lys Val Thr Ala Leu Thr Gln
           115                 120                 125
   Lys Pro Asp Val Tyr Ile Pro Glu Thr Leu Glu Pro Gly Gln Pro Val
       130                 135                 140
   Thr Val Ile Cys Val Phe Asn Trp Ala Phe Glu Glu Cys Pro Pro Pro
   145                 150                 155                 160
   Ser Phe Ser Trp Thr Gly Ala Ala Leu Ser Ser Gln Gly Thr Lys Pro
                   165                 170                 175
   Thr Thr Ser His Phe Ser Val Leu Ser Phe Thr Pro Arg Pro Gln Asp
               180                 185                 190
   His Asn Thr Asp Leu Thr Cys His Val Asp Phe Ser Arg Lys Gly Val
           195                 200                 205
   Ser Val Gln Arg Thr Val Arg Leu Arg Val Ala Tyr Ala Pro Arg Asp
       210                 215                 220
   Leu Val Ile Ser Ile Ser Arg Asp Asn Thr Pro Ala Leu Glu Pro Gln
   225                 230                 235                 240
   Pro Gln Gly Asn Val Pro Tyr Leu Glu Ala Gln Lys Gly Gln Phe Leu
                   245                 250                 255
   Arg Leu Leu Cys Ala Ala Asp Ser Gln Pro Pro Ala Thr Leu Ser Trp
               260                 265                 270
   Val Leu Gln Asn Arg Val Leu Ser Ser Ser His Pro Trp Gly Pro Arg
           275                 280                 285
   Pro Leu Gly Leu Glu Leu Pro Gly Val Lys Ala Gly Asp Ser Gly Arg
       290                 295                 300
   Tyr Thr Cys Arg Ala Glu Asn Arg Leu Gly Ser Gln Gln Arg Ala Leu
   305                 310                 315                 320
   Asp Leu Ser Val Gln Tyr Pro Pro Glu Asn Leu Arg Val Met Val Ser
                   325                 330                 335
   Gln Ala Asn Arg Thr Val Leu Glu Asn Leu Gly Asn Gly Thr Ser Leu
               340                 345                 350
   Pro Val Leu Glu Gly Gln Ser Leu Cys Leu Val Cys Val Thr His Ser
           355                 360                 365
   Ser Pro Pro Ala Arg Leu Ser Trp Thr Gln Arg Gly Gln Val Leu Ser
       370                 375                 380
   Pro Ser Gln Pro Ser Asp Pro Gly Val Leu Glu Leu Pro Arg Val Gln
   385                 390                 395                 400
   Val Glu His Glu Gly Glu Phe Thr Cys His Ala Arg His Pro Leu Gly
                   405                 410                 415
   Ser Gln His Val Ser Leu Ser Leu Ser Val His Tyr Ser Pro Lys Leu
               420                 425                 430
   Leu Gly Pro Ser Cys Ser Trp Glu Ala Glu Gly Leu His Cys Ser Cys
           435                 440                 445
   Ser Ser Gln Ala Ser Pro Ala Pro Ser Leu Arg Trp Trp Leu Gly Glu
       450                 455                 460
   Glu Leu Leu Glu Gly Asn Ser Ser Gln Asp Ser Phe Glu Val Thr Pro
   465                 470                 475                 480
   Ser Ser Ala Gly Pro Trp Ala Asn Ser Ser Leu Ser Leu His Gly Gly
                   485                 490                 495
   Leu Ser Ser Gly Leu Arg Leu Arg Cys Glu Ala Trp Asn Val His Gly
               500                 505                 510
   Ala Gln Ser Gly Ser Ile Leu Gln Leu Pro Asp Lys Lys Gly Leu Ile
   515 520 525
   Ser Thr Ala Phe Ser Asn Gly Ala Phe Leu Gly Ile Gly Ile Thr Ala
   530 535 540
   Leu Leu Phe Leu Cys Leu Ala Leu Ile Ile Met Lys Ile Leu Pro Lys
   545 550 555 560
   Arg Arg Thr Gln Thr Glu Thr Pro Arg Pro Arg Phe Ser Arg His Ser
   565 570 575
   Thr Ile Leu Asp Tyr Ile Asn Val Val Pro Thr Ala Gly Pro Leu Ala
   580 585 590
   Gln Lys Arg Asn Gln Lys Ala Thr Pro Asn Ser Pro Arg Thr Pro Leu
   595 600 605
   Pro Pro Gly Ala Pro Ser Pro Glu Ser Lys Lys Asn Gln Lys Lys Gln
   610 615 620
   Tyr Gln Leu Pro Ser Phe Pro Glu Pro Lys Ser Ser Thr Gln Ala Pro
   625 630 635 640
   Glu Ser Gln Glu Ser Gln Glu Glu Leu His Tyr Ala Thr Leu Asn Phe
   645 650 655
   Pro Gly Val Arg Pro Arg Pro Glu Ala Arg Met Pro Lys Gly Thr Gln
   660 665 670
   Ala Asp Tyr Ala Glu Val Lys Phe Gln
   675 680
<210> 59
   <211> 670
   <212> PRT
   <213> Homo sapiens
<400> 59
   Asn Lys Asp Pro Ser Tyr Ser Leu Gln Val Gln Arg Gln Val Pro Val
   1 5 10 15
   Pro Glu Gly Leu Cys Val Ile Val Ser Cys Asn Leu Ser Tyr Pro Arg
   20 25 30
   Asp Gly Trp Asp Glu Ser Thr Ala Ala Tyr Gly Tyr Trp Phe Lys Gly
   35 40 45
   Arg Thr Ser Pro Lys Thr Gly Ala Pro Val Ala Thr Asn Asn Gln Ser
       50                  55                  60
   Arg Glu Val Glu Met Ser Thr Arg Asp Arg Phe Gln Leu Thr Gly Asp
   65                  70                  75                  80
   Pro Gly Lys Gly Ser Cys Ser Leu Val Ile Arg Asp Ala Gln Arg Glu
                   85                  90                  95
   Asp Glu Ala Trp Tyr Phe Phe Arg Val Glu Arg Gly Ser Arg Val Arg
               100                 105                 110
   His Ser Phe Leu Ser Asn Ala Phe Phe Leu Lys Val Thr Ala Leu Thr
           115                 120                 125
   Lys Lys Pro Asp Val Tyr Ile Pro Glu Thr Leu Glu Pro Gly Gln Pro
       130                 135                 140
   Val Thr Val Ile Cys Val Phe Asn Trp Ala Phe Lys Lys Cys Pro Ala
   145                 150                 155                 160
   Pro Ser Phe Ser Trp Thr Gly Ala Ala Leu Ser Pro Arg Arg Thr Arg
                   165                 170                 175
   Pro Ser Thr Ser His Phe Ser Val Leu Ser Phe Thr Pro Ser Pro Gln
               180                 185                 190
   Asp His Asp Thr Asp Leu Thr Cys His Val Asp Phe Ser Arg Lys Gly
           195                 200                 205
   Val Ser Ala Gln Arg Thr Val Arg Leu Arg Val Ala Tyr Ala Pro Lys
       210                 215                 220
   Asp Leu Ile Ile Ser Ile Ser His Asp Asn Thr Ser Ala Leu Glu Leu
   225                 230                 235                 240
   Gln Gly Asn Val Ile Tyr Leu Glu Val Gln Lys Gly Gln Phe Leu Arg
                   245                 250                 255
   Leu Leu Cys Ala Ala Asp Ser Gln Pro Pro Ala Thr Leu Ser Trp Val
               260                 265                 270
   Leu Gln Asp Arg Val Leu Ser Ser Ser His Pro Trp Gly Pro Arg Thr
           275                 280                 285
   Leu Gly Leu Glu Leu Arg Gly Val Arg Ala Gly Asp Ser Gly Arg Tyr
       290                 295                 300
   Thr Cys Arg Ala Glu Asn Arg Leu Gly Ser Gln Gln Gln Ala Leu Asp
   305                 310                 315                 320
   Leu Ser Val Gln Tyr Pro Pro Glu Asn Leu Arg Val Met Val Ser Gln
                   325                 330                 335
   Ala Asn Arg Thr Val Leu Glu Asn Leu Gly Asn Gly Thr Ser Leu Pro
               340                 345                 350
   Val Leu Glu Gly Gln Ser Leu Arg Leu Val Cys Val Thr His Ser Ser
           355                 360                 365
   Pro Pro Ala Arg Leu Ser Trp Thr Arg Trp Gly Gln Thr Val Gly Pro
       370                 375                 380
   Ser Gln Pro Ser Asp Pro Gly Val Leu Glu Leu Pro Pro Ile Gln Met
   385                 390                 395                 400
   Glu His Glu Gly Glu Phe Thr Cys His Ala Gln His Pro Leu Gly Ser
                   405                 410                 415
   Gln His Val Ser Leu Ser Leu Ser Val His Tyr Pro Pro Gln Leu Leu
               420                 425                 430
   Gly Pro Ser Cys Ser Trp Glu Ala Glu Gly Leu His Cys Ser Cys Ser
           435                 440                 445
   Ser Gln Ala Ser Pro Ala Pro Ser Leu Arg Trp Trp Leu Gly Glu Glu
       450                 455                 460
   Leu Leu Glu Gly Asn Ser Ser Gln Gly Ser Phe Glu Val Thr Pro Ser
   465                 470                 475                 480
   Ser Ala Gly Pro Trp Ala Asn Ser Ser Leu Ser Leu His Gly Gly Leu
                   485                 490                 495
   Ser Ser Gly Leu Arg Leu Arg Cys Lys Ala Trp Asn Val His Gly Ala
               500                 505                 510
   Gln Ser Gly Ser Val Phe Gln Leu Leu Pro Gly Lys Leu Glu His Gly
           515                 520                 525
   Gly Gly Leu Gly Leu Gly Ala Ala Leu Gly Ala Gly Val Ala Ala Leu
       530                 535                 540
   Leu Ala Phe Cys Ser Cys Leu Val Val Phe Arg Val Lys Ile Cys Arg
   545 550 555 560
   Lys Glu Ala Arg Lys Arg Ala Ala Ala Glu Gln Asp Val Pro Ser Thr
   565 570 575
   Leu Gly Pro Ile Ser Gln Gly His Gln His Glu Cys Ser Ala Gly Ser
   580 585 590
   Ser Gln Asp His Pro Pro Pro Gly Ala Ala Thr Tyr Thr Pro Gly Lys
   595 600 605
   Gly Glu Glu Gln Glu Leu His Tyr Ala Ser Leu Ser Phe Gln Gly Leu
   610 615 620
   Arg Leu Trp Glu Pro Ala Asp Gln Glu Ala Pro Ser Thr Thr Glu Tyr
   625 630 635 640
   Ser Glu Ile Lys Ile His Thr Gly Gln Pro Leu Arg Gly Pro Gly Phe
   645 650 655
   Gly Leu Gln Leu Glu Arg Glu Met Ser Gly Met Val Pro Lys
   660 665 670
<210> 60
   <211> 577
   <212> PRT
   <213> Homo sapiens
<400> 60
   Lys Glu Gln Lys Asp Tyr Leu Leu Thr Met Gln Lys Ser Val Thr Val
   1 5 10 15
   Gln Glu Gly Leu Cys Val Ser Val Leu Cys Ser Phe Ser Tyr Pro Gln
   20 25 30
   Asn Gly Trp Thr Ala Ser Asp Pro Val His Gly Tyr Trp Phe Arg Ala
   35 40 45
   Gly Asp His Val Ser Arg Asn Ile Pro Val Ala Thr Asn Asn Pro Ala
   50 55 60
   Arg Ala Val Gln Glu Glu Thr Arg Asp Arg Phe His Leu Leu Gly Asp
   65 70 75 80
   Pro Gln Asn Lys Asp Cys Thr Leu Ser Ile Arg Asp Thr Arg Glu Ser
   85 90 95
   Asp Ala Gly Thr Tyr Val Phe Cys Val Glu Arg Gly Asn Met Lys Trp
               100                 105                 110
   Asn Tyr Lys Tyr Asp Gln Leu Ser Val Asn Val Thr Ala Ser Gln Asp
           115                 120                 125
   Leu Leu Ser Arg Tyr Arg Leu Glu Val Pro Glu Ser Val Thr Val Gln
       130                 135                 140
   Glu Gly Leu Cys Val Ser Val Pro Cys Ser Val Leu Tyr Pro His Tyr
   145                 150                 155                 160
   Asn Trp Thr Ala Ser Ser Pro Val Tyr Gly Ser Trp Phe Lys Glu Gly
                   165                 170                 175
   Ala Asp Ile Pro Trp Asp Ile Pro Val Ala Thr Asn Thr Pro Ser Gly
               180                 185                 190
   Lys Val Gln Glu Asp Thr His Gly Arg Phe Leu Leu Leu Gly Asp Pro
           195                 200                 205
   Gln Thr Asn Asn Cys Ser Leu Ser Ile Arg Asp Ala Arg Lys Gly Asp
       210                 215                 220
   Ser Gly Lys Tyr Tyr Phe Gln Val Glu Arg Gly Ser Arg Lys Trp Asn
   225                 230                 235                 240
   Tyr Ile Tyr Asp Lys Leu Ser Val His Val Thr Ala Leu Thr His Met
                   245                 250                 255
   Pro Thr Phe Ser Ile Pro Gly Thr Leu Glu Ser Gly His Pro Arg Asn
               260                 265                 270
   Leu Thr Cys Ser Val Pro Trp Ala Cys Glu Gln Gly Thr Pro Pro Thr
           275                 280                 285
   Ile Thr Trp Met Gly Ala Ser Val Ser Ser Leu Asp Pro Thr Ile Thr
       290                 295                 300
   Arg Ser Ser Met Leu Ser Leu Ile Pro Gln Pro Gln Asp His Gly Thr
   305                 310                 315                 320
   Ser Leu Thr Cys Gln Val Thr Leu Pro Gly Ala Gly Val Thr Met Thr
                   325                 330                 335
   Arg Ala Val Arg Leu Asn Ile Ser Tyr Pro Pro Gln Asn Leu Thr Met
               340                 345                 350
   Thr Val Phe Gln Gly Asp Gly Thr Ala Ser Thr Thr Leu Arg Asn Gly
           355                 360                 365
   Ser Ala Leu Ser Val Leu Glu Gly Gln Ser Leu His Leu Val Cys Ala
       370                 375                 380
   Val Asp Ser Asn Pro Pro Ala Arg Leu Ser Trp Thr Trp Gly Ser Leu
   385                 390                 395                 400
   Thr Leu Ser Pro Ser Gln Ser Ser Asn Leu Gly Val Leu Glu Leu Pro
                   405                 410                 415
   Arg Val His Val Lys Asp Glu Gly Glu Phe Thr Cys Arg Ala Gln Asn
               420                 425                 430
   Pro Leu Gly Ser Gln His Ile Ser Leu Ser Leu Ser Leu Gln Asn Glu
           435                 440                 445
   Tyr Thr Gly Lys Met Arg Pro Ile Ser Gly Val Thr Leu Gly Ala Phe
       450                 455                 460
   Gly Gly Ala Gly Ala Thr Ala Leu Val Phe Leu Tyr Phe Cys Ile Ile
   465                 470                 475                 480
   Phe Val Val Val Arg Ser Cys Arg Lys Lys Ser Ala Arg Pro Ala Val
                   485                 490                 495
   Gly Val Gly Asp Thr Gly Met Glu Asp Ala Asn Ala Val Arg Gly Ser
               500                 505                 510
   Ala Ser Gln Gly Pro Leu Ile Glu Ser Pro Ala Asp Asp Ser Pro Pro
           515                 520                 525
   His His Ala Pro Pro Ala Leu Ala Thr Pro Ser Pro Glu Glu Gly Glu
       530                 535                 540
   Ile Gln Tyr Ala Ser Leu Ser Phe His Lys Ala Arg Pro Gln Tyr Pro
   545                 550                 555                 560
   Gln Glu Gln Glu Ala Ile Gly Tyr Glu Tyr Ser Glu Ile Asn Ile Pro
                   565                 570                 575
   Lys